# EUROPÄISCHE PATENTSCHRIFT

(11) **EP 1 674 441 B1**
(45) Veröffentlichungstag und Bekanntmachung des Hinweises auf die Patenterteilung: **05.10.2011**
(21) Anmeldenummer: 05109526.3
(22) Anmeldetag: 13.10.2005
(51) Int. Cl.: C07C 45/50, C07C 47/02

(54) **Verfahren zur Hydroformylierung von Olefinen**
Process for the hydroformylation of olefins
Procédé pour l'hydroformylation d'olefines

(30) Priorität: 09.12.2004 DE 102004059293
(43) Veröffentlichungstag der Anmeldung: 28.06.2006
(73) Patentinhaber: Evonik Oxeno GmbH, 45772 Marl (DE)
(72) Erfinder: Lüken, Hans-Gerd, 45770, Marl (DE); Kaizik, Alfred, 45772, Marl (DE); Drees, Stefan, 48249, Dülmen (DE); Büschken, Wilfried, 45721, Haltern am See (DE); Droste, Wilhelm, 45770, Marl (DE)
(74) Vertreter: Hirsch, Hans-Ludwig

(56) Entgegenhaltungen:
- DE-A1- 10 034 360
- US-A- 4 658 068

## Beschreibung

Die vorliegende Erfindung betrifft ein Verfahren zur Herstellung von Aldehyden und/oder Alkoholen durch Hydroformylierung von Olefinen oder Olefingemischen in Gegenwart eines Kobalt-Katalysators, Abtrennen des Katalysators und gegebenenfalls anschließende Hydrierung, wobei in mindestens einer Hydroformylierungsstufe den Edukten ein Aldehyd zugefügt wird.

Höhere Aldehyde, insbesondere solche mit 7 bis 25 Kohlenstoffatomen, können bekanntlich durch katalytische Hydroformylierung (technisch meist als Oxo-Reaktion bezeichnet) der um ein Kohlenstoffatom ärmeren Olefine hergestellt werden. Die Aldehyde werden zum Beispiel als Synthesevorstufen, zur Erzeugung von Carbonsäuren und als Riechstoffe genutzt. Technisch werden sie oft durch katalytische Hydrierung in die entsprechenden Alkohole überführt, die u. a. als Zwischenprodukte für die Herstellung von Weichmachern und Detergenzien Einsatz finden.

Verfahren zur Hydroformylierung von Olefinen sind in der Literatur in großer Zahl beschrieben. Die Wahl des Katalysatorsystems und der optimalen Reaktionsbedingungen für die Hydroformylierung sind von der Reaktivität des eingesetzten Olefms abhängig. Der Einfluss der Struktur des eingesetzten Olefins auf dessen Reaktivität in der Hydroformylierung ist z. B. von J. FALBE, "New Syntheses with Carbon Monoxide ", Springer Verlag, 1980, Berlin, Heidelberg, New York, Seite 95 ff, beschrieben.

Als allgemeine Regel gilt, dass die Geschwindigkeit von Hydroformylierungsreaktionen bei konstanten Rahmenbedingungen mit steigender Kohlenstoffzahl und mit zunehmendem Verzweigungsgrad des Olefins abnimmt. So kann die Reaktionsgeschwindigkeit von linearen Olefinen die der verzweigten Isomeren um mehr als eine Zehnerpotenz übersteigen. Zusätzlich hat die Lage der Doppelbindung im Olefin einen entscheidenden Einfluss auf die Reaktivität. Olefine mit terminaler Doppelbindung reagieren deutlich schneller als Isomere mit der Doppelbindung im Innern des Moleküls. Die unterschiedliche Reaktivität von isomeren Octenen wurde zum Beispiel von B. L. HAYMORE, A. van HASSELT, R. BECK, Annals of the New York Acad. Sci., 1983, 415, 159-175 untersucht. Eine allgemeine Übersicht und weiterführende Literatur geben B. CORNILS, W. A. HERRMANN, "Applied Homogeneous Catalysis with Organometallic Compounds", Vol. 1&2, VCH, Weinheim, New York, 1996.

Technische Olefingemische, die als Edukte für die Hydroformylierungssynthese verwendet werden, enthalten oftmals Olefmisomere der verschiedensten Strukturen mit unterschiedlichen Verzweigungsgraden, unterschiedlicher Lage der Doppelbindung und zum Teil Olefine unterschiedlicher Molmassen. Dies gilt insbesondere für Olefingemische, die durch Di-, Tri- oder weitergehende Oligomerisierung von Olefinen mit 2 bis 8 C-Atomen oder anderen leicht zugänglichen höheren Olefinen bzw. durch Cooligomerisierung der genannten Olefine erhältlich sind. Als Beispiele für typische Olefingemische, die technisch für die Hydroformylierung relevant sind, seien Tri- und Tetrapropen sowie Di-, Tri- und Tetrabutene genannt.

Bei einer technisch durchgeführten Hydroformylierung ist es erwünscht, neben einem hohen Umsatz eine hohe Selektivität zu erreichen, um eine optimale Ausnutzung des Rohstoffes zu gewährleisten. Um einen hohen Umsatz zu erreichen, müssen bei langsam reagierenden Olefinen oft eine relativ lange Reaktionszeit und/oder höhere Reaktionstemperaturen in Kauf genommen werden. Reaktivere Olefine werden dagegen unter den gleichen Reaktionsbedingungen schon in weitaus kürzerer Zeit zu den Aldehyden umgesetzt. Bei der gemeinsamen Hydroformylierung von Gemischen von Olefinen unterschiedlicher Reaktivität führt dies dazu, dass man relativ lange Reaktionszeiten benötigt, um einen ausreichenden Umsatz auch der schwerer hydroformylierbaren Olefine zu erzielen. Die aus den leichter umsetzbaren Olefinen entstehenden Aldehyde werden jedoch relativ schnell gebildet und liegen dann neben den schwerer hydroformylierbaren Olefmen im Reaktor vor. Dies führt zu unerwünschten Neben- und Folgereaktionen der Aldehyde, z. B. zur Hydrierung, zu Kondensationsreaktionen sowie zur Bildung von Acetalen und Halbacetalen. Vor allem wegen der unterschiedlichen Reaktivität der Olefinisomeren ist es also schwierig, bei der Hydroformylierung hohe Umsätze und gleichzeitig hohe Selektivitäten zu erzielen.

Um Nebenreaktionen, insbesondere die des/der bei der Hydroformylierung entstehenden Aldehyds/Aldehyde, gering zu halten, werden bei technischen Verfahren die Olefinumsätze und somit die Aldehydkonzentrationen im Hydroformylierungsreaktor begrenzt. Die nicht umgesetzten Olefine werden nach Abtrennung der Hydroformylierungsprodukte in dem gleichen oder einen oder mehreren weiteren Hydroformylierungsreaktor(en) umgesetzt.

In DE 198 42 371 wird die Herstellung von Alkoholen mit 6 bis 25 C-Atomen durch einstufige Hydroformylierung der entsprechenden Olefine und Hydrierung der Hydroformylierungsprodukte beschrieben. Dabei wird im Hydroformylierungsreaktor im geraden Durchgang nur ein Teil des zugeführten Olefins umgesetzt. Der flüssige Reaktoraustrag wird vom Hydroformylierungskatalysator befreit und selektiv hydriert, d. h., die Aldehyde werden zu den entsprechenden Alkoholen, jedoch nicht die Olefine hydriert. Vom Austrag der Selektivhydrierung werden destillativ die Olefine abgetrennt und in den Hydroformylierungsreaktor zurückgeführt.

Verfahren zur Herstellung höherer Oxo-Alkohole, bei denen die Hydroformylierung in mindestens zwei Reaktoren durchgeführt werden und aus dem Hydroformylierungsaustrag durch Destillation die nicht umgesetzten Olefine abgetrennt werden und in einen Hydroformylierungsreaktor zurückgeführt werden, werden beispielsweise in DE 100 34 360, DE 198 42 368 und EP 1 057 803 beschrieben.

In DE 100 34 360 wird ein Verfahren zur mehrstufigen Kobalt- oder Rhodium-katalysierten Hydroformylierung von Olefinen mit 6 bis 24 Kohlenstoffatomen zu Alkoholen und/oder Aldehyden beschrieben, wobei die Olefine
a) in einem Hydroformylierungsschritt bis zu einem Umsatz von 20 bis 98 % hydroformyliert werden,
b) der Katalysator aus dem so erhaltenen flüssigen Reaktoraustrag entfernt wird,
c) das so erhaltene flüssige Hydroformylierungsgemisch in eine Leichtsiederfraktion, enthaltend Olefine und Paraffine und eine Sumpffraktion, enthaltend Aldehyde und/oder Alkohole getrennt wird,
die in der Leichtsiederfraktion enthaltenden Olefine in weiteren Verfahrensstufen, umfassend die Verfahrensschritte a, b und c umgesetzt werden
und die Sumpffraktionen der Verfahrensschritte c) aller Verfahrensstufen vereinigt werden.

Bevorzugt wird dieses Verfahren so ausgeübt, dass der flüssige Reaktoraustrag der Hydroformylierungsschritte a) eine homogene Flüssigphase ist. Die Kobalt- oder Rhodium-Katalysatoren werden bevorzugt so eingesetzt, dass sie homogen im flüssigen Reaktoraustrag der Hydroformylierungsschritte a) gelöst sind.

In DE 198 42 368 wird ein Verfahren zur Herstellung von höheren Oxo-Alkoholen aus Gemischen isomerer Olefine mit 5 bis 24 Kohlenstoffatomen durch zweistufige Hydroformylierung in Gegenwart eines Kobalt- oder Rhodium-Katalysators bei erhöhter Temperatur und erhöhtem Druck beschrieben, bei dem man das Reaktionsgemisch der ersten Hydroformylierungsstufe selektiv hydriert, das Hydrierungsgemisch in einer Destillation in rohen Alkohol und überwiegend aus Olefmen bestehende Leichtsieder trennt, diese in die zweite Hydroformylierungsstufe fährt, das Reaktionsgemisch der zweiten Hydroformylierungsstufe wiederum selektiv hydriert, das Hydriergemischgemisch in einer Destillation in rohen Alkohol und Leichtsieder trennt, den rohen Alkohol durch Destillation auf reinen Alkohol aufarbeitet und zumindest einen Teil der Leichtsieder zur Ausschleusung gesättigter Kohlenwasserstoffe abzieht.

In EP 1 057 803 wird ein zweistufiges Verfahren zur Herstellung von Alkoholen aus Olefmen oder Olefingemischen offengelegt. In diesem Verfahren wird in der ersten Reaktionsstufe das Einsatzolefin in Gegenwart eines Kobaltkatalysators zu 50 bis 90 % hydroformyliert. Nach der Abtrennung des Katalysators werden vom Reaktionsaustrag die nicht umgesetzten Olefine destillativ abgetrennt und die abgetrennten Olefine im zweiten Hydroformylierungsreaktor umgesetzt. Die Hydroformylierungsprodukte aus beiden Stufen können zu den entsprechenden Alkoholen hydriert werden. In beiden Reaktionsstufen wird als Katalysator Co₂(CO)₈ oder HCo(CO)₄ eingesetzt, der außerhalb der Hydroformylierungsreaktoren erzeugt wird. Aus dem Reaktionsgemisch der Hydroformylierung wird vor Weiterverarbeitung durch Extraktion mit einer Base der Kobaltkatalysator entfernt.

Bei den bekannten Verfahren zur Hydroformylierung von Olefinen oder Olefingemischen mit 6 bis 24 C-Atomen entstehen trotz Begrenzung des Olefmumsatzes in jedem Hydroformylierungsreaktor Nebenprodukte, wie beispielsweise Kondensationsprodukte der Aldehyde, Ether oder Paraffine.

Daher bestand die Aufgabe der vorliegenden Erfindung darin, ein Verfahren bereitzustellen, mit dem Olefine oder Olefingemische in größerer Ausbeute als nach herkömmlichen Verfahren zu Wertprodukten, wie Aldehyden, Alkoholen und deren Formiaten, umgesetzt werden können. Die Wertprodukte können anschließend zu dem/den Alkohol(en) hydriert werden.

Überraschenderweise wurde nun gefunden, dass die Ausbeute an Wertprodukten bei einer ein- oder mehrstufigen Hydroformylierung, bei der zumindest in einem Reaktor ein unmodifizierter Kobaltkomplex als Katalysator wirkt, erhöht werden kann, wenn in diesem Reaktor bezogen auf das eingespeiste Olefin 0,5 bis 20 Mol-% Aldehyd (also das Produkt der Hydroformylierung) eingeleitet wird.

Dieser Befund ist unerwartet, da wie oben beschrieben, die Selektivität für die Bildung von Wertprodukten mit zunehmenden Umsatz der Olefine und somit steigender Aldehydkonzentration im Reaktor abnimmt. Daher wäre zu erwarten gewesen, dass bei Einsatz eines reinen Olefins oder eines reinen Gemisches mehrerer Olefine eine höhere Selektivität für die Bildung von Wertprodukten erhalten wird als bei einem Einsatzprodukt, das neben Olefinen auch Aldehyd(e) enthält. Umso überraschender ist es daher, dass bei der Hydroformylierung unter Verwendung eines unmodifizierten Kobaltkomplexes als Katalysator die Gegenwart von Aldehyd(en) im Einsatzolefin in einem Bereich von 0,5 bis 20 Mol-%, bezogen auf die Olefin(e), die Selektivität der Wertproduktbildung erhöht.

Gegenstand der vorliegenden Erfindung ist deshalb ein Verfahren zur katalytischen Hydroformylierung von Olefmen mit 6 bis 24 C-Atomen, welches dadurch gekennzeichnet ist, dass die Hydroformylierung in zumindest einer Stufe, also in einer oder mehreren Stufe(n) durchgeführt wird, wobei in zumindest einer dieser Stufen, die in Gegenwart eines unmodifizierten Kobaltkomplexkatalysators durchgeführt wird, als Edukt ein Gemisch eingesetzt wird, welches zumindest ein Olefin und ein Aldehyd mit 7 bis 25 C-Atomen aufweist, wobei das molare Verhältnis von Aldehyd zu Olefm von 0,005 zu 1 bis 0,2 zu 1 beträgt.

Ebenfalls Gegenstand der vorliegenden Erfindung ist ein Eduktgemisch, welches geeignet ist zum Einsatz im erfindungsgemäßen Verfahren, welches zumindest ein Olefin mit 6 bis 24 C-Atomen und ein Aldehyd mit 7 bis 25 C-Atomen aufweist, wobei das molare Verhältnis von Aldehyd zu Olefin von 0,005 zu 1 bis 0,2 zu 1 beträgt.

Im Sinne der vorliegenden Erfmdung werden unter unmodifizierten oder nicht modifizierten Komplexen, Metallkomplexe, insbesondere des Kobalts oder des Rhodiums, verstanden, deren Liganden ausgewählt sind aus Kohlenmonoxid, Wasserstoff, Olefm, Alkylreste, Alkenylreste, Acylreste oder Alkoxyreste, wobei als Liganden keine Verbindungen, die Elemente der fünften Hauptgruppe des Periodensystems der Elemente (N, P, As, Sb; Bi) enthalten, vorhanden sind.

Im Sinne der vorliegenden Erfindung sind unter Eduktgemischen solche Mischungen zu verstehen, die zu Beginn einer Reaktion in einem Reaktor vorliegen oder die durch Einleiten in den Reaktor erhalten werden. Die im Eduktgemisch enthaltenen Verbindungen können dabei einzeln oder bereits als Gemisch in den Reaktor geleitet werden.

Die vorliegende Erfindung hat den Vorteil, dass bei der Hydroformylierung von höheren Olefinen die Ausbeute auf einfache Weise erhöht werden kann und gegebenenfalls zusätzlich Energie eingespart werden kann. Der Vorteil kann sowohl bei neu zu errichtenden Anlagen genutzt werden als auch bei schon in Betrieb befindlichen Anlagen. Beispielsweise können bei einer bestehenden Hydroformylierungsanlage, in der in mindestens zwei hintereinandergeschalteten Reaktoren hydroformyliert wird, die nicht umgesetzten Olefine destillativ abgetrennt und in den nächsten Reaktor eingespeist werden und der zweite oder folgende Reaktor einen unmodifizierten Kobaltkomplex als Katalysator enthält, der im gleichen Reaktor simultan mit der Hydroformylierung aus einer wässrigen Kobaltsalzlösung erzeugt wird, allein durch Änderung der Destillationsbedingungen in der Olefinabtrennungskolonne eine Erhöhung der Ausbeute an Aldehyden bzw. Alkoholen erreicht werden. Durch eine Verringerung des Rücklaufverhältnis kann im Destillat das gewünschte Olefin/Aldehyd-Verhältnis eingestellt werden. Gleichzeitig verringert sich der Olefingehalt des Sumpfproduktes. Durch die Senkung des Rücklaufverhältnis kann außerdem Energie eingespart werden. Ein zusätzlicher Kapitaleinsatz ist zur Umrüstung von bestehenden Verfahren auf das erfindungsgemäße Verfahren nicht erforderlich.

Das erfindungsgemäße Verfahren wird nachfolgend beispielhaft beschrieben, ohne dass die Erfindung auf die beispielhaften Ausführungsformen beschränkt sein soll. Sind nachfolgend Bereiche, allgemeine Formeln oder Verbindungsklassen angegeben, so sollen diese nicht nur die entsprechende Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur katalytischen Hydroformylierung von Olefinen mit 6 bis 24 C-Atomen, zeichnet sich dadurch aus, dass die Hydroformylierung in ein oder mehreren Stufen durchgeführt wird, wobei in zumindest einer dieser Stufen, die in Gegenwart eines unmodifizierten Kobaltkomplexkatalysators durchgeführt wird, als Edukt ein Gemisch eingesetzt wird, welches zumindest ein Olefm, insbesondere ein Olefin mit 6 bis 24 C-Atomen, und ein Aldehyd mit 7 bis 25 C-Atomen aufweist, wobei das molare Verhältnis von Aldehyd (bzw. Molsumme aller vorhandenen Aldehyde) zu Olefin (bzw. Molsumme aller vorhandener Olefme) von 0,005 zu 1 bis 0,2 zu 1, vorzugsweise von 0,01 zu 1 bis 0,1 zu 1 und besonders bevorzugt 0,05 zu 1 bis 0,07 zu 1 beträgt. Besonders bevorzugt weist das Eduktgemisch Aldehyde und Olefine auf, wobei die Olefine im Mittel ein Kohlenstoffatom weniger aufweisen als die Aldehyde. Wird Di-n-Buten hydroformyliert liegt das molare Verhältnis von Aldehyd zu Olefin vorzugsweise im Bereich von 0,01 zu 1 bis 0,07 zu 1. Eine Hydroformylierungsstufe, die in Gegenwart eines unmodifizierten Kobaltkomplexkatalysators durchgeführt wird und bei der als Edukt ein Gemisch eingesetzt wird, welches zumindest ein Olefin und ein Aldehyd mit 7 bis 25 C-Atomen aufweist, wobei das molare Verhältnis von Aldehyd zu Olefin von 0,005 zu 1 bis 0,2 zu 1 wird nachfolgend auch als OA-Hydroformylierungsstufe bezeichnet.

Als Aldehyd zur Herstellung des Eduktgemisches kann jedweder Aldehyd mit 7 bis 25 C-Atomen eingesetzt werden. Bevorzugt werden zur Herstellung des Eduktgemisches solche Aldehyde eingesetzt, die im Schnitt ein Kohlenstoffatom mehr aufweisen als die eingesetzten Olefme. Besonders bevorzugt werden zur Herstellung des Eduktgemisches solche Aldehyde eingesetzt, die als Produkt einer Hydroformylierung, insbesondere als Produkt einer Hydroformylierungsstufe des erfmdungsgemäßen Verfahrens erhalten werden. Die in der jeweiligen Hydroformylierungsstufe eingesetzten Aldehyde können aus anderen Hydroformylierungsstufen, aber auch aus der gleichen Hydroformylierungsstufe stammen.

Zur Herstellung des Aldehyd und Olefin aufweisenden Eduktgemisches kann z. B. ein Destillat, das Aldehyde und Olefine enthält und das aus einem Hydroformylierungsgemisch durch Destillation abgetrennt wurde, eingesetzt werden. Es kann aber auch direkt ein Hydroformylierungsgemisch oder ein Hydroformylierungsgemisch, aus dem zunächst der Katalysator entfernt wurde, zur Herstellung des Eduktgemisches aus Aldehyd und Olefin eingesetzt werden. Ebenso ist es möglich ein Destillat, das Aldehyde und Olefine enthält und das aus einem Hydroformylierungsgemisch abgetrennt wurde, und ein Hydroformylierungsgemisch oder ein Hydroformylierungsgemisch, aus dem zunächst der Katalysator entfernt wurde (entkatalysiertes Hydroformylierungsgemisch), zur Herstellung des Eduktgemisches aus Aldehyd und Olefin einzusetzen. Die Hydroformylierungsgemische oder die Teile davon können wiederum aus irgendeiner im Verfahren betriebenen Hydroformylierungsstufe stammen, wobei bei solchen Hydroformylierungsgemischen, die aus einer Stufe stammen, die mit einem Rhodiumkatalysator betrieben wird, zumindest der Katalysator zunächst aus dem Hydroformylierungsgemisch abgetrennt wird, bevor sie, nach einer optionalen weiteren Auftrennung, zur Herstellung des Eduktgemisches der OA-Hydroformylierungsstufe eingesetzt werden.

Zur Herstellung des Eduktgemisches mit den oben genannten Verhältnissen von Olefin(en) zu Aldehyd(en) gibt es z. B. die folgenden Möglichkeiten:
1. Die destillative Aufarbeitung eines entkatalysierten, olefinhaltigen Hydroformylierungsgemisches kann durch die Wahl der Destillationsbedingungen so eingestellt werden, dass die Leichtsiederfraktion neben Olefinen auch Aldehyde enthält. Durch Variation der Destillationsbedingungen, insbesondere des Rücklaufverhältnis, kann direkt das gewünschte Aldehyd/Olefin-Verhältnis eingestellt werden. Dieser Strom oder eine Teilmenge davon kann ohne Zumischung von Olefinen und/oder Aldehyden in einer OA-Hydroformylierungsstufe als Edukt verwendet werden.
2. Durch Mischen eines wie in 1. erhaltene Gemisches in geeigneten Verhältnissen mit Frischolefin und/oder einer olefinhaltigen (Leichtsieder-)Fraktion, die aus einer Hydroformylierungsstufe erhalten worden ist, zu einem Gemisch welches die genannten Verhältnisse an Olefin und Aldehyd aufweist.
3. Durch Mischen eines Hydroformylierungsgemisches, dass einen unmodifizierten Kobaltkomplex oder einen Vorläufer davon enthält, in geeigneten Verhältnissen mit Frischolefin und/oder einer olefinhaltigen (Leichtsieder-)Fraktion, die aus einer Hydroformylierungsstufe erhalten worden ist und die optional Aldehyde enthalten kann, zu einem Gemisch, welches die genannten Verhältnisse an Olefin und Aldehyd aufweist.

Unter Hydroformylierungsstufen werden im Rahmen der vorliegenden Erfindung einzelne oder parallel geschaltete Hydroformylierungsreaktoren oder in Serie geschaltete Hydroformylierungsreaktoren, bei denen die Edukte nur dem ersten Reaktor der Serie zugeführt werden, verstanden. Das Verfahren kann entweder nur in einer Hydroformylierungsstufe oder in zwei oder mehr Hydroformylierungsstufen durchgeführt werden. Wird das Verfahren in mehreren Stufen durchgeführt, so wird jeweils zumindest ein Teil des Hydroformylierungsproduktes, welcher nicht umgesetzte Olefme enthält, gegebenenfalls nach Abtrennung des Katalysators und/oder einer teilweisen oder vollständigen Abtrennung der Aldehyde in die nächste Hydroformylierungsstufe eingespeist.

Es kann vorteilhaft sein, wenn neben einer Hydroformylierungsstufe, die in Gegenwart eines unmodifizierten Kobaltkomplexkatalysators durchgeführt wird und bei der ein Eduktgemisch, welches zumindest Olefine(e) und Aldehyd(e) aufweist, eingesetzt wird, mindestens eine weitere Hydroformylierungsstufe vorhanden ist, in welcher ein unmodifizierter oder modifizierter Kobaltkatalysator oder ein modifizierten oder unmodifizierter Rhodiumkomplex als Katalysator eingesetzt wird. Der Einsatz einer zusätzlichen Hydroformylierungsstufe, die einen Rhodiumkatalysator aufweist, kann insbesondere dann vorteilhaft sein, wenn die Ausbeute des Hydroformylierungsverfahrens im Vordergrund steht, unabhängig davon, wie hoch der Anteil der terminal hydroformylierten Produkte ist. Verwendet man beispielsweise bei Einsatz des als Di-n-buten bekannten Dimerisierungsgemisches von n-Butenen als Olefin in der ersten Stufe einen unmodifizierten Kobalt- und in der oder den folgenden Stufen einen unmodifizierten Rhodiumkatalysator, so verbessert sich die Ausbeute, während der Anteil der terminal hydroformylierten Produkte etwas zurückgeht.

Es kann aber auch vorteilhaft sein, wenn in allen im erfindungsgemäßen Verfahren vorhandenen Hydroformylierungsstufen Kobaltkomplexe, insbesondere unmodifizierte Kobaltkomplexe als Katalysatoren eingesetzt werden. Der ausschließliche Einsatz von Kobaltkatalysatoren in erfindungsgemäßen Verfahren, bei denen mehr als eine Hydroformylierungsstufe vorhanden ist, kann insbesondere dann vorteilhaft sein, wenn das Produkt einen möglichst hohen Anteil an terminal hydroformylierten Olefinen aufweisen soll. So erzielt man beispielsweise beim Einsatz des als Di-n-buten bekannten Gemisches von Dimerisierungsprodukten der n-Butene als Olefm einen sehr hohen Anteil an terminal hydroformyliertem Produkt bei zufriedenstellender Ausbeute, wenn bei einem Zweistufenverfahren in beiden Stufen unmodifizierte Kobaltkatalysatoren einsetzt.

Sind in dem erfindungsgemäßen Verfahren ausschließlich Hydroformylierungsstufen oder mehrere Hydroformylierungsstufen vorhanden, in denen Kobaltkatalysatoren eingesetzt werden, so kann es insbesondere vorteilhaft sein, wenn in mehreren, bevorzugt in allen Hydroformylierungsstufen, in denen Kobaltkomplexe als Katalysatoren eingesetzt werden, als Edukt ein Gemisch eingesetzt wird, welches zumindest ein Olefin und ein Aldehyd aufweist.

Die in den Hydroformylierungsstufen eingesetzten unmodifizierten Kobaltkomplexe können gleichzeitig mit der Hydroformylierung durch Umsetzung einer wässrigen Kobaltsalzlösung mit Synthesegas in der Hydroformylierungsstufe erzeugt werden oder vorgefertigte Katalysatoren sein. Bevorzugt werden unmodifizierte Kobaltkatalysatoren eingesetzt, die gleichzeitig mit der Hydroformylierung durch Umsetzung einer wässrigen Kobaltsalzlösung mit Synthesegas in der Hydroformylierungsstufe erzeugt werden. Insbesondere wenn die Hydroformylierung einstufig durchgeführt wird, wird vorzugsweise ein unmodifizierter Kobaltkomplex, der während der Hydroformylierung bevorzugt im gleichen Reaktor durch Umsetzung einer wässrigen Kobaltsalzlösung mit Synthesegas erzeugt wird, als Katalysator verwendet. Bei Verfahren, bei denen mehrere Hydroformylierungsstufen vorhanden sind, können Kobalt- und Rhodiumkatalysatoren mit oder ohne Komplex-stabilisierende Zusätze bzw. Liganden, wie z. B. organische Phosphine oder Phosphite, eingesetzt werden, mit der Maßgabe, dass zumindest eine Hydroformylierungsstufe als OA-Hydroformylierungsstufe durchgeführt wird. Ansonsten hängt die Wahl des Katalysators und der Reaktionsbedingungen (Konzentration des Katalysators, Temperatur, Druck, Verweilzeit) unter anderem von der Anzahl der Kohlenstoffatome und der Zusammensetzung der Ausgangsolefine ab.

In dem erfindungsgemäßen Verfahren können als Edukte Olefine oder Gemische von Olefinen mit 6 bis 24 Kohlenstoffatomen, vorzugsweise mit 6 bis 20 Kohlenstoffatomen und besonders bevorzugt mit 8 bis 20 Kohlenstoffatomen eingesetzt werden. Die Gemische können Olefine mit end- und/oder innenständige C-C-Doppelbindungen aufweisen. Die Gemische können Olefine mit gleicher, ähnlicher (± 2) oder deutlich unterschiedlicher (>±2) Kohlenstoffatomzahl (C-Zahl) aufweisen oder aus diesen bestehen. Als Olefine, die entweder in reiner Form, in einem Isomerengemisch oder in einem Gemisch mit weiteren Olefinen anderer C-Zahl als Edukt eingesetzt werden können, seien beispielhaft genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2- oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Olefine sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefinen, wie n-Butenen, Isobuten oder Propen. Andere ebenfalls gut geeignete Edukte sind Oligomere aus C₅-Olefinen.

Für die Oligomerisierung von Butenen zu im Wesentlichen C₈-Olefinen enthaltenden Gemischen gibt es im Prinzip drei Verfahrensvarianten. Lange bekannt ist die Oligomerisierung an sauren Katalysatoren, wobei technisch z. B. Zeolithe oder Phosphorsäure auf Trägern eingesetzt werden. Hierbei werden Isomerengemische von verzweigten Olefinen erhalten, die im Wesentlichen Dimethylhexene darstellen (WO 92/ 13818). Ein ebenfalls weltweit ausgeübtes Verfahren ist die Oligomerisierung mit löslichen Ni-Komplexen, bekannt als DIMERSOL-Verfahren (B. CORNILS, W. A. HERRMANN, *"Applied Homogeneous*

Catalysis with Organicmetallic Compounds"; Vol. 1&2, VCH, Weinheim, New York **1996**). Die dritte Verfahrensvariante ist die Oligomerisierung an Nickel-Festbett-Katalysatoren; das Verfahren hat Eingang in die Literatur als OCTOL-Prozess (Hydrocarbon Process., Int. Ed. (1986) 65 (2. Sect.1) Seiten 31 -33) gefunden und wird z. B. in EP 0 395 857 beschrieben. Für die erfindungsgemäße Herstellung eines C₉-Alkoholgemischs, das sich insbesondere für die Darstellung von Weichmachern eignet, wird bevorzugt ein C₈-Olefingemisch, das aus linearen Butenen nach dem OCTOL-Prozess gewonnen worden ist, eingesetzt.

In den Hydroformylierungsstufen wird vorzugsweise ein Synthesegas eingesetzt, bei dem das molare Verhältnis von Kohlenmonoxid zu Wasserstoff bevorzugt von 1 : 4 bis 4 : 1, besonders bevorzugt von 1 : 2 bis 2 : 1, ganz besonders bevorzugt von 1 : 1,2 bis 1,2 : 1 beträgt. Insbesondere wird ein Synthesegas eingesetzt, bei dem ein in etwa stöchiometrischen Verhältnis von Kohlenmonoxid und Wasserstoff vorliegt.

Die Temperaturen und die Drücke in den Hydroformylierungsstufen des erfindungsgemäßen Verfahrens können, je nach Katalysator und Olefingemisch, in weiten Grenzen variieren. Da bei einer mehrstufigen Ausführung des Verfahrens in der ersten Stufe bevorzugt die reaktiveren Olefine reagieren, werden in den folgenden Hydroformylierungsstufen bevorzugt energischere Reaktionsbedingungen hinsichtlich Temperatur, Katalysatormenge und Verweilzeit eingestellt.

Die optimalen Bedingungen können, je nach Zielsetzung, von Fall zu Fall variieren: So können zum Beispiel die insgesamt erzielte Raum-Zeit-Ausbeute, die Erhöhung der Selektivität oder die gewünschten Produkteigenschaften ein Optimierungskriterium sein. In der Regel wird die Zusammensetzung des Eduktolefins und die Wahl der Katalysatorsysteme und/oder der Reaktionsbedingungen den Ausschlag geben, welche der möglichen Ausführungsformen des erfindungsgemäßen Verfahrens die ökonomisch optimale ist. Die Optimierung kann der Fachmann auf einfache Weise durch Simulationsrechnungen bestimmen.

Bevorzugt wird das erfmdungsgemäße Verfahren so durchgeführt, dass beim Vorhandensein von mehreren Hydroformylierungsstufen in den einzelnen Hydroformylierungsstufen Umsätze von 20 bis 98 %, insbesondere von 40 bis 80 %, besonders bevorzugt 50 bis 75 % erhalten werden(jeweils im geraden Durchgang).

Rhodium- und Kobalt-katalysierte Hydroformylierungsverfahren unterscheiden sich meistens durch ihre Betriebsparameter. Der Hauptunterschied liegt jedoch in der grundsätzlich unterschiedlichen Katalysatorabtrennung und -rückführung. Im Folgenden werden die beiden Verfahren getrennt erläutert.

### Kobalt-katalysierte Hydroformylierung

Im erfindungsgemäßen Verfahren wird in mindestens einer Hydroformylierungsstufe ein unmodifizierter Kobaltkomplex als Katalysator eingesetzt. In anderen Stufen können bevorzugt unmodifizierte und/oder modifizierte Kobaltkomplexe oder aber Rhodium-Komplexe als Katalysator verwendet werden. Die unmodifizierten Kobaltkatalysatoren können außerhalb des Hydroformylierungsreaktor hergestellt oder bevorzugt innerhalb des Hydroformylierungsreaktor simultan zu der Hydroformylierung der Olefine durch Umsetzung einer wässrigen Kobaltsalzlösung mit Synthesegas erzeugt werden.

Ein Verfahren, bei dem der unmodifizierte Kobaltkatalysator (HCo(CO)₄ und/oder Co₂(Co)₄) während der Hydroformylierung im Hydroformylierungsreaktor erzeugt wird, wird beispielsweise in DE 196 54 340 beschrieben. Nach diesem bevorzugt im erfindungsgemäßen Verfahren als Hydroformylierungsstufe eingesetzten Verfahren werden die Ausgangsstoffe, also eine Kobaltsalzlösung, das organische Eduktgemisch und das Synthesegas, gleichzeitig, vorzugsweise mit Hilfe einer Mischdüse, im Gleichstrom von unten in den Reaktor eingebracht.

Als Kobaltverbindungen zur Herstellung des Katalysators bzw. der Kobaltsalzlösung können bevorzugt Kobaltsalze, wie Formiate, Acetate oder Salze von Carbonsäuren, die wasserlöslich sind, verwendet werden. Besonders bevorzugt wird Kobaltacetat eingesetzt. Die Kobaltsalze können als wässrige Lösung eingesetzt werden, die vorzugsweise einen Kobalt-Gehalt von 0,5 bis 3 Massen-%, bevorzugt von 0,8 bis 1,8 Massen-%, gerechnet als Metall, aufweisen. Die eingesetzte Kobaltsalzlösung kann eine frisch angesetzte Lösung sein und/oder aber eine Kobaltsalzlösung, die bei der Katalysatorabtrennung aus dem Hydroformylierungsaustrag einer Hydroformylierungsstufe erhalten wird.

Die im Hydroformylierungsreaktor gewünschte Wassermenge kann mit der Kobaltsalzlösung eingebracht werden, deren Konzentration in einem weiten Bereich variiert werden kann. Es ist jedoch auch möglich, neben der Kobaltsalzlösung zusätzliches Wasser einzuspeisen.

Es hat sich als vorteilhaft erwiesen, bei dem Kobalt-katalysierten Verfahren der Dosierung der Edukte in den Hydroformylierungsreaktor eine große Bedeutung beizumessen. Zur Einspeisung der Edukte in den Hydroformylierungsreaktor sollte dieser eine Dosiervorrichtung aufweisen, die eine gute Phasenvermischung und die Erzeugung einer möglichst hohen Phasenaustauschfläche gewährleistet. Eine solche Dosiervorrichtung kann z. B. eine Mischdüse sein. Ferner kann es vorteilhaft sein, den Reaktorraum der Hydroformylierungsreaktoren durch den Einbau von 1 bis 10, vorzugsweise von 2 bis 4, senkrecht zur Fließrichtung des Reaktanden- und Produktstromes angeordneten Lochblechen zu unterteilen. Durch diese Reaktorkaskadierung wird die Rückvermischung gegenüber der einfachen Blasensäule stark vermindert und das Strömungsverhalten dem eines Rohreaktors angenähert. Durch diese verfahrenstechnische Maßnahme kann erreicht werden, dass sowohl die Ausbeute als auch die Selektivität der Hydroformylierung verbessert werden.

Genauere Angaben zur Durchführen der Kobalt-katalysierten Hydroformylierung aber auch spezielle Ausführungsarten des Durchführens der Hydroformylierung in der Hydroformylierungsstufe können z. B. DE 199 39 491 A1 und DE 101 35 906 entnommen werden. So wird gemäß DE 199 39 491 aus dem unteren Teil des Reaktors ein Teilstrom der flüssigen Mischphase (wässrige Kobaltsalzlösung/organische Phase) abgezogen und an einer höheren Stelle des Reaktors eingespeist. Nach DE 101 35 906 wird im Hydroformylierungsreaktor der Stand der wässrigen Phase konstant gehalten, wobei die Konzentration an Kobaltverbindungen (berechnet als metallisches Kobalt) in dieser wässrigen Sumpfphase im Bereich von 0,4 bis 1,7 Massen-% liegt.

In einer anderen Ausführungsart des erfmdungsgemäßen Verfahrens wird der fertige Katalysator in Form einer Katalysatorlösung, die Co₂(CO)₈ gelöst in den Einsatzolefmen enthält, oder bei höheren Temperaturen in flüssiger Form in den Hydroformylierungsreaktor eingespeist. Zu dieser im Reaktor vorgelegten Katalysatorlösung, wird Olefin, Katalysator, Synthesegas und optional Wasser und Aldehyd dem Reaktor zugeführt. Wasser kann dabei schon vor dem Reaktor in das Olefin dispergiert werden, beispielsweise durch Verwendung eines Statikmischers. Es ist jedoch auch möglich, alle Komponenten erst im Reaktor zu vermischen.

Die Kobalt-katalysierte Hydroformylierung wird vorzugsweise bei Temperaturen von 100 bis 250 °C, bevorzugt 140 bis 210 °C und/oder bei einem Druck von 10 bis 40 MPa, bevorzugt von 20 bis 30 MPa durchgeführt. Das Volumenverhältnis von Kohlenmonoxid zu Wasserstoff im eingesetzten Synthesegas beträgt vorzugsweise von 2 zu 1 bis 1 zu 2, bevorzugt von 1,5 zu 1 bis 1 zu 1,5 und besonders bevorzugt von 1 zu 1 bis 1 zu 1,5. Das Synthesegas wird vorzugsweise im Überschuss, zum Beispiel bis zu dem Dreifachen der stöchiometrischen Menge in Bezug auf das Olefin, eingesetzt. Im flüssigen Hydroformylierungsaustrag beträgt die Konzentration an Kobaltverbindungen (berechnet als metallisches Kobalt) üblicherweise von 0,01 bis 0,5 Massen-%, insbesondere 0,02 bis 0,08 Massen-% (bezogen auf die Summe aus organischer und wässriger Phase).

Durch die unterschiedlichen Möglichkeiten der Wasserzugabe ist der Wassergehalt im Eintrag des Hydroformylierungsreaktors nur schwierig zu bestimmen. Im Folgenden werden daher Angaben über den Wassergehalt im Austrag des Reaktors gemacht, wobei der Wassergehalt im Reaktoraustrag praktisch gleichbedeutend mit dem Wassergehalt der flüssigen Phase während der Reaktion ist. Die Wasserkonzentrationen in den flüssigen Hydroformylierungsausträgen der Kobalt-katalysierten Hydroformylierungsstufen können von 0,1 bis 10 Massen-%, insbesondere von 0,5 bis 5 Massen-% betragen. Sind mehrere Kobalt-katalysierte Hydroformylierungsstufen vorhanden, so können die Wassergehalte der Hydroformylierungsausträge der einzelnen Stufen gleich oder unterschiedlich sein. Bevorzugt ist der Wassergehalt so groß, dass das Wasser in den flüssigen Hydroformylierungsausträgen homogen gelöst ist.

Bei Verfahren mit mehreren Hydroformylierungsstufen können in den Reaktoren, in denen Kobaltverbindungen als Katalysator eingesetzt werden, gleiche oder unterschiedliche Bedingungen eingestellt werden. Besonders bevorzugt wird bei der Durchführung des erfindungsgemäßen Verfahrens in mehreren Hydroformylierungsstufen in der ersten Hydroformylierungsstufe, in der die reaktiveren Olefine umgesetzt werden, eine Temperaturen von 140 bis 195 °C, vorzugsweise von 160 bis 185 °C eingestellt. In dieser Verfahrensstufe werden bevorzugt Olefm-Umsätze von 20 bis 95 %, vorzugsweise von 50 bis 80 % angestrebt. In der oder den nachfolgenden Hydroformylierungsstufen, in denen die weniger reaktiven Olefine hydroformyliert werden, wird die Hydroformylierung bevorzugt bei höheren Temperaturen als den in der ersten Hydroformylierungsstufe verwendeten durchgeführt. Ebenfalls ist es möglich, die Hydroformylierung der weniger reaktiven Olefme unter Verwendung eines anderen Katalysators, insbesondere eines modifizierten Kobalt oder eines modifizierten oder unmodifizierten Rhodiumkatalysators durchzuführen.

### Entkobaltung

Der Hydroformylierungsaustrag einer mit einem unmodifizierten Kobaltkomplex als Katalysator betriebenen Hydroformylierungsstufe wird nach Verlassen des Hydroformylierungsreaktors bevorzugt auf einen Druck von 1,0 bis 3,0 MPa entspannt und in eine flüssige und eine gasförmige Phase getrennt. Die flüssige Phase wird anschließend dem eigentlichen Entkobaltungsschritt zugeführt. Im Entkobaltungsschritt wird die flüssige Phase des Hydroformylierungsaustrags in Gegenwart von sauren, wässrigen Kobalt(II)-salzlösungen ("Prozesswasser") mit sauerstoffhaltigen Gasen, insbesondere Luft oder Sauerstoff bei Temperaturen von 90 bis 160 °C umgesetzt und so oxidativ von Kobalt-Carbonylkomplexen befreit. Die hydroformylierungsaktiven Kobaltcarbonylkomplexe werden so unter Bildung von Kobalt(II)-salzen zerstört. Die Entkobaltungsverfahren sind gut bekannt und in der Literatur ausführlich, so z. B von J. FALBE, in "New Syntheses with Carbon Monoxide", Springer Verlag (1980), Berlin, Heidelberg, New York, Seite 158 ff., beschrieben. Die als Prozesswasser eingesetzte Lösung weist vorzugsweise einen pH-Wert von 1,5 bis 4,5 auf. Der Kobaltgehalt des eingesetzten Prozesswasser entspricht vorzugsweise nahezu (Abweichung kleiner ± 20 %, vorzugsweise kleiner ± 10 % und bevorzugt kleiner ± 5 %) dem Kobaltgehalt des im Hydroformylierungsschritt eingesetzten Katalysator- bzw. Katalysatorvorläufergemisches und weist besonders bevorzugt einen Kobaltgehalt von 0,8 bis 2,0 Massen-% auf.

Die Entkobaltung kann in einem mit Füllkörpern, wie z. B. Raschig-Ringen, befüllten Druckbehälter, in dem vorzugsweise eine möglichst hohe Phasenaustauschfläche erzeugt wird, durchgeführt werden. Das durch die Entkobaltung erhaltene Produktgemisch wird, z. B. in einem nachgeschalteten Trennbehälter, in eine wässrige und eine praktisch kobaltfreie organische Phase getrennt. Die wässrige Phase, das "Prozesswasser", die das zurückextrahierte, aus der organischen Phase wiedergewonnene Kobalt in Form von Kobaltacetat/formiat aufweist, wird ganz oder nach Ausschleusung eines geringen Anteils in eine Hydroformylierungsstufe, vorzugsweise in die jeweilige Hydroformylierungsstufe zurückgeführt und vorzugsweise als Ausgangsstoff für die in-situ Herstellung der Kobalt-Katalysatorkomplexe verwendet.

Optional kann vor der Rückführung des Prozesswassers in den Hydroformylierungsreaktor ein Teil der überschüssigen Ameisensäure entfernt werden. Dies kann beispielsweise durch Destillation erfolgen. Eine andere Möglichkeit besteht darin, einen Teil der Ameisensäure zu zersetzen, beispielsweise katalytisch, wie dies in DE 100 09 207 beschrieben.

Weiterhin ist es möglich, aus der bei der Entkobaltung anfallenden Kobaltsalzlösung durch Vorcarbonylierung den eigentlichen Hydroformylierungskatalysator (Co₂(CO)₈ und/oder HCo(CO)₄) herzustellen und in eine Hydroformylierungsstufe zurückzuführen.

Die organische Phase enthält unter anderem nicht umgesetzte Olefine, Aldehyde, Alkohole, Ameisensäureester und Hochsieder sowie Spuren an Kobaltverbindungen. Die organischen Phasen, die aus dem flüssigen Hydroformylierungsgemisch einer oder mehrere Hydroformylierungsstufen nach Entfernen des Katalysators erhalten werden können, werden vorzugsweise einer Aufarbeitung zugeführt, bei der die Leichtsieder, vorzugsweise die nicht umgesetzten Olefinen von den Wertprodukten (Aldehyd, Alkohol, Formiate) getrennt werden. Diese Aufarbeitung wird nachfolgend beschrieben.

### Aufarbeitung

Die Abtrennung der Olefme aus dem Hydroformylierungsaustrag erfolgt vorzugsweise nach Abtrennung des Katalysators und/oder einer gegebenenfalls vorhandenen Katalysatorphase und kann beispielsweise durch Destillation oder Wasserdampfdestillation erfolgen. Die Destillation wird vorzugsweise so durchgeführt, dass eine Kopffraktion (Leichtsiederfraktion) erhalten wird, die die nicht umgesetzten Olefine, die durch Hydrierung von Olefmen entstandenen Paraffine und gelöstes Wasser aufweist. Es kann vorteilhaft sein, wenn zumindest ein Teil der abgetrennten Olefine bzw. der Kopffraktion in eine oder mehrere Hydroformylierungsstufen zurückgeführt wird. Wenn diese Fraktion als aldehydhaltige Fraktion zur Erzeugung des Eduktgemisches oder direkt als Eduktgemisch eingesetzt werden soll, so müssen die Destillationsbedingungen entsprechend so gewählt werden, dass ein gewünschter Anteil an Aldehyden im Kopfprodukt anfällt. Diese Fraktionen können allein oder nach Abmischen mit einem Olefin aufweisenden Gemisch in eine OA-Hydroformylierungsstufe geleitet werden, wobei diese z. B. eine vorangegangene Stufe, dieselbe Stufe oder eine nachfolgende Hydroformylierungsstufe sein kann. Die nachfolgende Hydroformylierungsstufe kann insbesondere die letzte Hydroformylierungsstufe sein.

Neben einer Aufarbeitung in einer einfachen Destillationskolonne ist es auch möglich die Aufarbeitung in einer Destillationskolonne mit einem oder mehreren Seitenabzügen durchzuführen. Aus dem oder den Seitenabzügen können Fraktionen entnommen werden, die Aldehyde und Olefine aufweisen. Bei dieser Ausführungsform der Aufarbeitung kann als Kopfprodukt eine Fraktion erhalten werden, die frei oder zumindest nahezu frei an Aldehyden ist. Durch die Durchführung der Aufarbeitung in einer Destillationskolonne mit einem oder mehreren Seitenabzügen können also zwei verschiedene olefinhaltige Fraktionen erhalten werden, wodurch, insbesondere beim Vorhandensein von mehreren Hydroformylierungsstufen, die Flexibilität der Verwendung der olefinhaltigen Fraktionen steigt. So kann die Kopffraktion z. B. ganz oder teilweise einer Hydroformylierungsstufe zugeführt werden, in der ein Rhodiumkatalysator eingesetzt wird, während die Fraktion aus dem Seitenabzug zur Herstellung eines Eduktgemisches für eine OA-Hydroformylierungsstufe oder direkt als Eduktgemisch eingesetzt werden kann. Es versteht sich von selbst, dass auch die Kopffraktion ganz oder teilweise zur Herstellung eines Eduktgemisches verwendet werden kann.

### Rhodiumkatalysierte Hydroformylierung

Weist das erfindungsgemäße Verfahren Hydroformylierungsstufen auf, in denen modifizierte und/oder unmodifizierte Rhodium-Katalysatoren eingesetzt werden, so können diese Hydroformylierungsverfahren gemäß dem Stand der Technik durchgeführt werden, wie dies z. B. in EP 0 213 639, EP 0 214 622, WO 2004/020380 oder WO 2004/024661 beschrieben wird. Sind mehrere Hydroformylierungsstufen vorhanden, in denen Rhodiumkatalysatoren eingesetzt werden, so können die verwendeten Rhodiumkatalysatoren gleich oder unterschiedlich sein. Nach Abtrennung des Katalysators und einer gegebenenfalls vorhandenen Katalysatorphase kann die weitere Aufarbeitung des Hydroformylierungsgemisches wie oben für die Hydroformylierungsstufen, die in Gegenwart von Kobaltkatalysatoren durchgeführt werden, beschrieben, erfolgen.

### Hydrierung

Wenn nicht der Aldehyd sondern der entsprechende Alkohol die eigentliche Zielverbindung ist, kann der Aldehyd zum Alkohol hydriert werden. Zum Zweck der Hydrierung kann der vorzugsweise von dem Katalysator und einer gegebenenfalls vorhandenen wässrigen Phase getrennte Austrag aus der Hydroformylierungsstufe einer Hydrierungsstufe zugeführt werden. Vor der Zuführung kann es vorteilhaft sein, gegebenenfalls im Austrag noch vorhandene Olefine ebenfalls abzutrennen. Dies kann beispielsweise durch Destillation erfolgen. Soll auf die Abtrennung verzichtet werden, so werden die gegebenenfalls vorhandenen Olefine entweder in der Hydrierung zu Paraffinen hydriert oder aber die Hydrierung muss so selektiv ausgeführt werden, dass die gegebenenfalls vorhandenen Olefine in der Hydrierstufe nicht oder nur zu einem geringen Anteile (< 10 %) hydriert werden.

Sind mehrere Hydroformylierungsstufen im erfindungsgemäßen Verfahren vorhanden, so kann jede Stufe, nur eine Stufe oder nur einige der Stufen eine Abtrennung der Olefine und/oder eine Hydrierungsstufe aufweisen. Insbesondere kann es vorteilhaft sein, wenn nur eine Abtrennung oder nur eine Hydrierung vorhanden ist. In diesem Fall werden die Hydrierausträge gesammelt einer Abtrennung und/oder eine Hydrierungsstufe zugeführt. Eine weitere Möglichkeit der Ausführung der Hydrierung besteht darin, dass bei mehreren Hydroformylierungsstufen in allen bis auf die letzte Stufe eine Abtrennung der Olefine erfolgt und die Aldehydfraktionen hydriert werden und in der letzten Stufe der von Katalysator und einer gegebenenfalls vorhandenen wässrigen Phase getrennt Hydroformylierungsaustrag direkt hydriert wird. Die Aldehydfraktionen können gemeinsam in einer Hydrierstufe oder aber in unterschiedlichen Hydrierstufen hydriert werden. Besonders bevorzugt wird zumindest ein Teil des Hydroformylierungsgemisches der letzten Hydroformylierungsstufe, vorzugsweise nach Abtrennung von Katalysator und gegebenenfalls vorhandener wässriger Phase hydriert.

Es kann vorteilhaft sein, vor der Hydrierung geringe Mengen an Katalysatorresten bzw. deren Folgeprodukte in einem weiteren Schritt abzutrennen. Ein Verfahren zur Abtrennung von Resten von Kobaltverbindungen durch Extraktion mit Wasser wird beispielsweise in DE 102 27 995 beschrieben.

Zur Hydrierung können z. B. Nickel-, Kupfer-, Kupfer/Nickel-, Kupfer/Chrom-, Kupfer/Chrom/Nickel-, Zink/Chrom-, Nickel/Molybdän-Katalysatoren verwenden werden. Die Katalysatoren können trägerfrei sein, oder die hydrieraktiven Stoffe bzw. ihre Vorläufer können auf Trägern, wie beispielsweise Siliziumdioxid oder Aluminiumoxid, aufgebracht sein.

Bevorzugte Katalysatoren, an denen die Hydroformylierungsgemische hydriert werden, enthalten, jeweils 0,3 bis 15 Massen-% Kupfer und Nickel sowie als Aktivatoren 0,05 bis 3,5 Massen-% Chrom und vorteilhaft 0,01 bis 1,6 Massen-%, vorzugsweise 0,02 bis 1,2 Massen-% einer Alkalikomponente auf einem Trägermaterial, vorzugsweise Aluminiumoxid und Siliziumdioxid. Die Mengenangaben beziehen sich auf den noch nicht reduzierten Katalysator. Die Alkalikomponente ist optional. Dieser Katalysator ist insbesondere als Katalysator zur selektiven Hydrierung der Aldehyde geeignet. Weitere Details zur selektiven Hydrierung können DE 198 42 370 entnommen werden.

Die Katalysatoren werden vorzugsweise in einer Form eingesetzt, in der sie einen geringen Strömungswiderstand bieten, z. B. in Form von Granalien, Pellets oder Formkörpern, wie Tabletten, Zylindern, Strangextrudate oder Ringen. Sie werden vorzugsweise vor ihrem Einsatz, z. B. durch Erhitzen im Wasserstoffstrom, aktiviert.

Die Hydrierung, wird vorzugsweise als Flüssigphasenhydrierung durchgeführt. Bevorzugt erfolgt die Hydrierung bei einem Gesamtdruck von 0,5 bis 50 MPa, besonders bevorzugt bei einem Druck von 1,5 bis 10 MPa. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen bei der Hydrierung in flüssiger oder gasförmiger Phase vorzugsweise im Bereich von 120 bis 220 °C, bevorzugt von 140 bis 180 °C. Beispiele für solche Hydrierungen die in der Hydrierungsstufe eingesetzt werden können sind z. B. in den Patentanmeldungen DE 198 42 369 und DE 198 42 370 beschrieben. Optional kann die Hydrierung unter Zusatz von Wasser erfolgen. Ein Flüssigphasenverfahren zur Hydrierung von Hydroformylierungsgemischen in Gegenwart von Wasser wird beispielsweise in DE 102 41 266 offengelegt.

Der Austrag aus der oder den Hydrierstufen kann in einer oder mehreren Destillationen zu Reinalkohol aufgearbeitet werden.

Das erfindungsgemäße Verfahren kann diskontinuierlich oder bevorzugt kontinuierlich durchgeführt werden.

Nachfolgend werden einige Verfahrensvarianten des erfindungsgemäßen Verfahrens, unter anderem auch einige Verfahrensvarianten für die mehrstufige Hydroformylierung exemplarisch als Zweistufenprozess dargestellt, beschrieben. Es sei jedoch betont, dass die hierbei beschriebenen Verfahrensweisen sinngemäß auch für Verfahren mit mehr als zwei Hydroformylierungsstufen gelten. Die nachfolgend exemplarisch aufgeführten Ausführungsformen des erfindungsgemäßen Verfahren sollen außerdem nicht als Einschränkung des erfindungsgemäßen Verfahrens auf diese Ausführungsformen verstanden werden.

### Ausführungsform 1

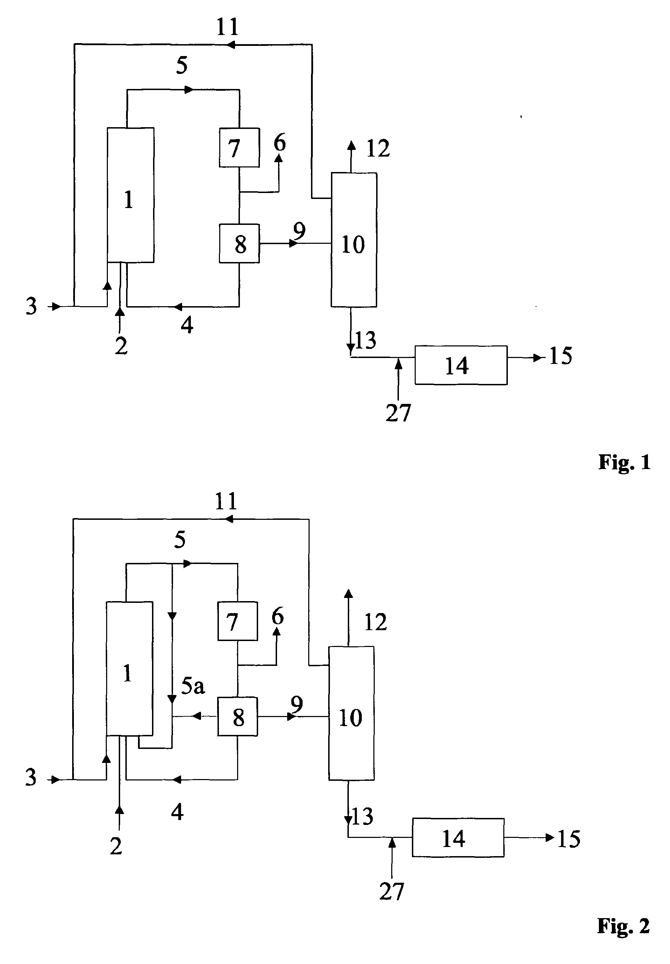

Eine Variante der Ausführungsform 1 des erfindungsgemäßen Verfahrens ist als Blockschema in Fig. 1 wiedergegeben. Bei dieser Variante wird die Hydroformylierung in einer Hydroformylierungsstufe in einem Reaktor durchgeführt. Der Katalysator ist ein unmodifizierter Kobaltkomplex, der gleichzeitig mit der Hydroformylierung durch Umsetzung einer wässrigen Kobaltsalzlösung mit Synthesegas erzeugt wird. In den Hydroformylierungsreaktor 1 werden das Einsatzolefin(gemisch) 3, Rückolefm 11, Synthesegas (Kohlenstoffmonoxid und Wasserstoff) 2 und eine wässrige Lösung einer Kobaltverbindung 4 eingespeist. Das so erhaltene Hydroformylierungsgemisch 5 wird auf 1,5 bis 3,0 MPa entspannt und nach der mit Wasser und Luft durchgeführten Entkobaltung 7 in der Katalysatorabtrennung 8 von Kobaltlösung 4 befreit. Vor der Katalysatorabtrennung 8 wird überschüssiges Synthesegas 6 abgezogen. Die Kobaltsalze enthaltende wässrige Phase 4 wird, gegebenenfalls nach Ausschleusung eines kleinen Teilstroms und nach Ergänzung durch frischen Katalysator, in den Hydroformylierungsreaktor 1 zurückgeführt. Mit Katalysator seien hier auch Vorstufen von Katalysatoren, z. B. Kobalt(II)salzlösungen, bezeichnet. Die vom Katalysator befreite organische Phase 9 wird in einer Trennstufe 10 in eine Leichtsiederfraktion 11, die Olefin und Aldehyde enthält, eine praktisch aldehydfreie Fraktion 12, die zur Abtrennung von Paraffinen und sonstigen leichsiedenden Nebenprodukten ausgeschleust wird, und in Rohaldehyd 13 getrennt. Der Rohaldehyd 13 kann in der Hydriereinheit 14 mit Wasserstoff 27 zu den entsprechenden Alkoholen 15 hydriert werden, die in einer nicht dargestellten Destillationseinheit auf Reinalkohol aufgearbeitet werden können.

### Ausführungsform 2

Auch in dieser Ausführungsform des erfindungsgemäßen Verfahrens erfolgt die Hydroformylierung wiederum in nur einer Hydroformylierungsstufe in einem Reaktor. Der Katalysator wird wiederum im Hydroformylierungsreaktor gleichzeitig zur Hydroformylierung durch Umsetzung von Synthesegas mit einer wässrigen Kobaltsalzlösung erzeugt. Ausführungsform 2 unterscheidet sich von Ausführungsform 1 dadurch, dass die gewünschte Aldehydmenge ganz oder teilweise durch Rückführung eines Teils des flüssigen Hydroformylierungsaustrags eingebracht wird.

Eine Variante der Ausführungsform 2 des erfindungsgemäßen Verfahrens ist als Blockschema in Fig. 2 wiedergegeben. In den Hydroformylierungsreaktor 1 werden das Einsatzolefin(gemisch) 3, gegebenenfalls Rückolefin 11, Synthesegas (Kohlenstoffmonoxid und Wasserstoff) 2, eine wässrige Lösung einer Kobaltverbindung 4 und Hydroformylierungsaustrag 5a, mit oder ohne Katalysator, eingespeist. Das so erhaltene Hydroformylierungsgemisch 5 wird auf 1,5 bis 3 MPa entspannt und nach der mit Wasser und Luft durchgeführten Entkobaltung 7 in der ersten Katalysatorabtrennung 8 von Kobaltverbindungen 4 befreit. Vor der Katalysatorabtrennung 8 wird überschüssiges Synthesegas 6 abgezogen. Die Kobaltsalze enthaltende wässrige Phase 4 wird, gegebenenfalls nach Ausschleusung eines kleinen Teilstroms und nach Ergänzung durch frischen Katalysator, in den ersten Hydroformylierungsreaktor 1 zurückgeführt. Mit Katalysator seien hier auch Vorstufen von Katalysatoren, z. B. Kobalt(II)salzlösungen, bezeichnet. Die vom Katalysator befreite organische Phase 9 wird in einer Trennstufe 10 in eine Kohlenwasserstofffraktion 11, die Olefin und optional Aldehyde enthält, eine praktisch aldehydfreie Fraktion 12, die zur Abtrennung von Paraffinen und sonstigen leichsiedenden Nebenprodukten ausgeschleust wird, und in Rohaldehyd 13 getrennt. Wenn mit dem Destillat kein Aldehyd in den Reaktor zurückgeführt wird, kann optional Strom 11 und 12 zusammen als ein Strom abgezogen werden, woraus ein Ausschleusestrom abgetrennt wird. Der Rohaldehyd 13 kann in der Hydriereinheit 14 mit Wasserstoff 27 zu den entsprechenden Alkoholen hydriert werden. Die in einer nicht dargestellten Destillationseinheit auf Reinalkohol aufgearbeitet werden können.

### Ausführungsform 3

In der beispielhaften Ausführungsform 3 wird das Verfahren mit zwei Hydroformylierungsstufen mit jeweils einem Hydroformylierungsreaktor durchgeführt, wobei zumindest im zweiten Hydroformylierungsreaktor ein unmodifizierter Kobaltkomplex, der gleichzeitig mit der Hydroformylierung durch Umsetzung einer wässrigen Kobaltsalzlösung mit Synthesegas erzeugt werden kann, als Katalysator verwendet wird.

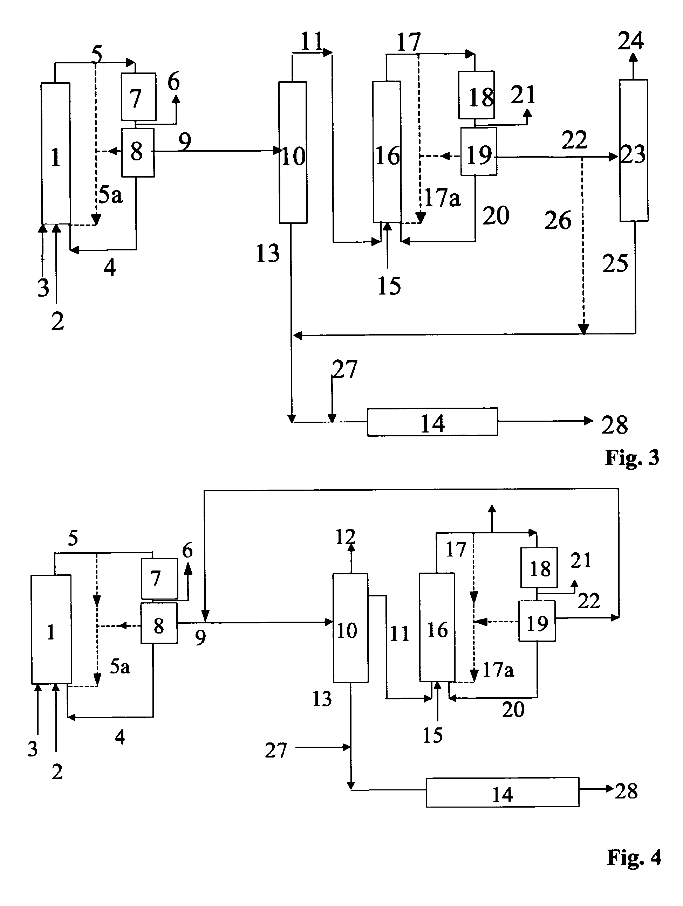

Eine Variante der Ausführungsform 3 des erfindungsgemäßen Verfahrens ist als Blockschema in Fig. 3 wiedergegeben. Im ersten Hydroformylierungsreaktor 1 werden das Olefingemisch 3, das Synthesegas 2 (Kohlenmonoxid und Wasserstoff) sowie eine Katalysatorlösung 4 eingespeist. Das so erhaltene Hydroformylierungsgemisch 5 wird entspannt und nach der mit Wasser und Luft durchgeführten Entkobaltung 7 in der ersten Katalysatorabtrennung 8 von Kobaltverbindungen 4 befreit. Vor der Katalysatorabtrennung 8 wird überschüssiges Synthesegas 6 abgezogen. Die den Katalysator enthaltene Phase 4 wird, gegebenenfalls nach Ausschleusung eines kleinen Teilstroms und nach Ergänzung durch frischen Katalysator, in den ersten Hydroformylierungsreaktor 1 zurückgeführt. Mit Katalysator seien hier auch Vorstufen von Katalysatoren, z. B. Kobalt(II)salzlösungen, bezeichnet. Die vom Katalysator befreite organische Phase 9 wird in einer Trennstufe 10 in eine Leichtsiederfraktion 11, die überwiegend aus nicht umgesetzten Olefinen und Aldehyden im gewünschten Verhältnis besteht, und Rohaldehyd 13 getrennt. Die Leichtsieder 11, Synthesegas 15 und eine wässrige Lösung einer Kobaltverbindung 20 werden in den zweiten Hydroformylierungsreaktor 16 eingebracht. Das Hydroformylierungsgemisch 17 aus dem zweiten Hydroformylierungsreaktor 16 wird auf 1,5 bis 3 MPa entspannt und nach der mit Wasser und Luft durchgeführten Entkobaltung 18 in der zweiten Katalysatorabtrennung 19 vom Katalysator 20 befreit. Vor der Katalysatorabtrennung 19 wird überschüssiges Synthesegas 21 abgezogen. Der abgetrennte Katalysator 20 wird, gegebenenfalls nach Ausschleusung eines kleinen Teilstroms und nach Ergänzung durch frischen Katalysator, in den zweiten Hydroformylierungsreaktor 16 zurückgeführt. Das entkatalysierte Hydroformylierungsgemisch 22 kann in der Trennstufe 23 in eine Leichtsiederfraktion 24, die überwiegend aus gesättigten Kohlenwasserstoffen besteht, und Rohaldehyd 25 aufgetrennt werden. Gegebenenfalls kann ein Teil der Leichtsiederfraktion 24 in einen der Reaktoren 16 oder 1 zurückgefahren werden. (Leitungen in Fig. 3 nicht dargestellt). Die aldehydhaltigen Fraktionen 13 und 25 können einzeln oder wie in Fig. 3 dargestellt gemeinsam einer Hydrierung 14 zugeführt werden, in denen die Aldehyde mit Wasserstoff 27 zu Alkoholen 28 hydriert werden, der optional in einer nicht dargestellten Destillation auf reinen Alkohol aufgearbeitet werden kann.

Eine weitere Ausgestaltung dieser Ausführungsform kann darin bestehen, dass das entkobaltete Hydroformylierungsgemisch 22, ohne Auftrennung in der Trennstufe 23, zusammen mit dem Rohaldehyd 13 aus der ersten Hydroformylierungsstufe der Hydriereinheit 14 zugeführt wird (Leitung 26).

Optional kann in Ausführungsform 3 durch Rückführung von katalysatorfreien oder Katalysator enthaltenden Hydroformylierungsaustrag 17a des zweiten Hydroformylierungsreaktors 16 Aldehyd in den zweiten Hydroformylierungsreaktor 16 eingespeist werden. Im Grenzfall kann mit Strom 17a die gewünschte Aldehydmenge in den zweiten Reaktor 16 eingebracht werden, sodass der Strom 11 praktisch keinen Aldehyd enthält bzw. enthalten muss.

Wird auch im ersten Reaktor ein unmodifizierter Kobaltkomplex, der im Hydroformylierungsreaktor gleichzeitig mit der Hydroformylierung durch Umsetzung einer wässrigen Kobaltsalzlösung mit Synthesegas erzeugt wird, als Katalysator verwendet, so kann es zweckmäßig sein, einen Teil des katalysatorfreien oder des Katalysator enthaltenen Hydroformylierungsaustrags 5a in den ersten Hydroformylierungsreaktor 1 rückzuführen. Ebenfalls könnte es vorteilhaft sein, einen Teil des aldehydhaltigen Stroms 11 (über eine nicht gezeichnete Leitung) in den ersten Hydroformylierungsreaktor zurückzuführen.

### Ausführungsform 4

In einer weiteren Ausführungsform 4 des erfindungsgemäßen Verfahrens sind ebenfalls zwei Hydroformylierungsstufen vorhanden, wobei wiederum zumindest im zweiten Hydroformylierungsreaktor ein unmodifizierter Kobaltkomplex, der gleichzeitig mit der Hydroformylierung durch Umsetzung einer wässrigen Kobaltsalzlösung mit Synthesegas erzeugt werden kann, verwendet wird. Der Katalysator für den ersten Reaktor ist frei wählbar. Der Hautunterschied zu Ausführungsform 3 besteht darin, dass die beiden entkatalysierten Hydroformylierungsausträge zusammen aufgearbeitet werden.

In Fig. 4 ist ein Blockschema einer Variante der Ausführungsform 4 des erfindungsgemäßen Verfahrens dargestellt. Im ersten Hydroformylierungsreaktor 1 werden das Olefingemisch 3, das Synthesegas 2 (Kohlenmonoxid und Wasserstoff) sowie eine Katalysatorlösung 4 eingespeist. Das so erhaltene Hydroformylierungsgemisch 5 wird entspannt und nach der mit Wasser und Luft durchgeführten Entkobaltung 7 in der ersten Katalysatorabtrennung 8 von Kobaltverbindungen 4 befreit. Vor der Katalysatorabtrennung 8 wird überschüssiges Synthesegas 6 abgezogen. Die den Katalysator enthaltene Phase 4 wird, gegebenenfalls nach Ausschleusung eines kleinen Teilstroms und nach Ergänzung durch frischen Katalysator, in den ersten Hydroformylierungsreaktor 1 zurückgeführt. Mit Katalysator seien hier auch Vorstufen von Katalysatoren, z. B. Kobalt(II)salzlösungen, bezeichnet. Die entkatalysierte organische Phase 9 wird in die Trennstufe 10 geleitet. Dort wird sie zusammen mit dem entkobalteten Hydroformylierungsgemisch 22 aus dem zweiten Hydroformylierungsreaktor 16 in eine Fraktion 11, die die nicht umgesetzten Olefme und Aldehyd enthält, eine Leichtsiederfraktion 12, in der Paraffine und sonstige Leichtsieder angereichert sind, und Rohaldehyd 13 getrennt. Die Leichtsiederfraktion 12 wird ausgeschleust. Die Kohlenwasserstofffraktion 11 wird zusammen mit Synthesegas 15 sowie einer wässrigen Lösung einer Kobaltverbindung 20 in den zweiten Hydroformylierungsreaktor 16 geführt. Das Hydroformylierungsgemisch 17 wird entspannt und nach der mit Wasser und Luft durchgeführten Entkobaltung 18 in einer zweiten Katalysatorabtrennung 19 vom Katalysator 20 befreit. Vor der Katalysatorabtrennung 19 wird das überschüssige Synthesegas 21 abgezogen. Der abgetrennte Katalysator 20 wird, gegebenenfalls nach Ausschleusung eines kleinen Teilstroms und nach Ergänzung durch frischen Katalysator, in den zweiten Hydroformylierungsreaktor 16 zurückgeführt. Das entkobaltete zweite Hydroformylierungsgemisch 22 wird mit dem Hydroformylierungsgemisch 9 der ersten Stufe, wie bereits erwähnt, in die Trennstufe 10 eingespeist. Der Rohaldehyd 13 kann in der Hydriereinheit 14 mit Wasserstoff 27 zum Rohalkohol 28 hydriert werden. Dieser Alkohol kann in einer nicht dargestellten Destillation auf den reinen Alkohol aufgearbeitet werden.

Optional kann in Ausführungsform 4 durch Rückführung von katalysatorfreien oder Katalysator enthaltenen Hydroformylierungsaustrag (gestrichelt gezeichnete Leitungen 17a) des zweiten Hydroformylierungsreaktors 16 Aldehyd in den zweiten Hydroformylierungsreaktor 16 eingespeist werden. Im Grenzfall kann mit Strom 17a die gewünschte Aldehydmenge in den zweiten Reaktor 16 eingebracht werden, sodass der Strom 11 praktisch keinen Aldehyd enthalten braucht.

Wird auch im ersten Reaktor ein unmodifizierter Kobaltkomplex, der im Hydroformylierungsreaktor gleichzeitig mit der Hydroformylierung durch Umsetzung einer wässrigen Kobaltsalzlösung mit Synthesegas erzeugt wird, als Katalysator verwendet, so kann es zweckmäßig sein, einen Teil des katalysatorfreien oder des Katalysator enthaltenen Hydroformylierungsaustrags (gestrichelt gezeichnete Leitungen 5a) in den ersten Hydroformylierungsreaktor 1 rückzuführen. Ebenfalls könnte es vorteilhaft sein, einen Teil des Aldehyd-haltigen Stroms 11 (über eine nicht gezeichnete Leitung) in den ersten Hydroformylierungsreaktor zurückzuführen.

### Ausführungsform 5

Auch die nachfolgend beschriebene Ausführungsform 5 des erfindungsgemäßen Verfahrens wird ebenfalls mit zwei Hydroformylierungsstufen durchgeführt. In beiden Reaktoren wirken unmodifizierte Kobaltkomplexe als Katalysator. Zumindest in einem Reaktor, insbesondere in dem zweiten Reaktor, wird der Katalysator während der Hydroformylierung durch Umsetzung einer wässrigen Kobaltsalzlösung mit Synthesegas erzeugt. Eine ganz besonders bevorzugte Ausführung dieser Variante besteht darin, in beiden Reaktoren den aktiven Kobaltkomplex während der Hydroformylierung durch Umsetzung einer Kobaltsalzlösung mit Synthesegas herzustellen.

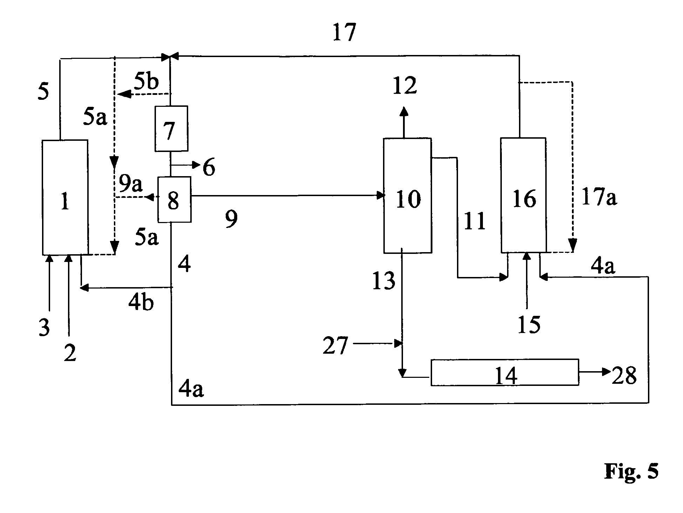

Ein Blockschema einer Variante der Ausführungsform 5 des erfindungsgemäßen Verfahrens ist in Fig. 5 dargestellt. In den ersten Hydroformylierungsreaktor 1 werden das Olefingemisch 3, das Synthesegas 2 (Kohlenmonoxid und Wasserstoff) sowie eine wässrige Lösung einer Kobaltverbindung 4b eingespeist. Das so erhaltene Hydroformylierungsgemisch 5 wird zusammen mit dem Hydroformylierungsgemisch 17 aus dem zweiten Hydroformylierungsreaktor 16 als vereinigte Hydroformylierungsausträge auf vorzugsweise 1,5 bis 3,0 MPa entspannt und nach der mit Wasser und Luft durchgeführten Entkobaltung 7 in der Katalysatorabtrennung 8 vom Katalysator 4 befreit. Vor der Katalysatorabtrennung 8 wird das überschüssige Synthesegas 6 abgezogen. Man erhält ein Gemisch 9, das die gebildeten Aldehyde, Alkohole und nicht umgesetzte Olefine enthält. Der Katalysator 4 wird, gegebenenfalls nach Ausschleusung einer Teilmenge und Ergänzung durch frischen Katalysator, in die beiden Teilströme 4b und 4a aufgeteilt. Teilstrom 4b wird in den ersten Hydroformylierungsreaktor 1 und Teilstrom 4a in den zweiten Hydroformylierungsreaktor 16 zurückgefahren. Der entkobaltete Hydroformylierungsaustrag 9 wird in der Trennstufe 10 in die Fraktion 11, die hauptsächlich aus Olefmen und Aldehyden besteht, eine praktisch aldehydfreie Leichtsiederfraktion 12, in der Paraffine und sonstige leichtsiedende Nebenprodukte angereichert sind, und den Rohaldehyd 13 aufgetrennt. Die Leichtsiederfraktion 12 wird ausgeschleust. Die Fraktion 11 wird zusammen mit Synthesegas 15 und einer wässriger Lösung einer Kobaltverbindung 4a in den zweiten Hydroformylierungsreaktor 16 eingeleitet. Der Rohaldehyd 13 kann in der Hydriereinheit 14 mit Wasserstoff 27 zum Rohalkohol 28 hydriert werden. Dieser kann wiederum in einer nicht dargestellten Destillation auf reinen Alkohol aufgearbeitet werden.

Optional kann in Variante 5 durch Einspeisung von Katalysator enthaltendem Hydroformylierungsaustrag 17a des zweiten Reaktors 16, durch Einspeisung eines Katalysator enthaltenden Gemisches beider Reaktoren (Leitung nicht gezeichnet, Entnahme vor oder in der Vorrichtung 7) oder Einspeisung des entkatalysierten Gemisches 9 beider Reaktoren (Leitung nicht gezeichnet) oder durch Einspeisung von zwei oder drei dieser Ströme Aldehyd in den zweiten Hydroformylierungsreaktor 16 eingebracht werden. Im Grenzfall kann/können mit diesem Strom/mit diesen Strömen die gewünschte Aldehydmenge in den zweiten Reaktor 16 eingetragen werden, sodass Strom 11 praktisch keinen Aldehyd enthalten braucht.

Optional kann in den ersten Reaktor 1 das Katalysator enthaltenden Hydroformylierungsgemisch 5a aus dem ersten Reaktor 1, das Katalysator enthaltenden Gemisch 5b der Hydroformylierungsausträge beider Reaktoren oder das entkatalysierte Gemisch 9a der beiden Hydroformylierungsausträge oder irgendeine Kombination dieser Ströme eingeleitet werden, um ein bestimmtes Olefin/Aldehyd-Verhältnis im Zufluss des ersten Reaktors einzustellen. Ebenfalls könnte es vorteilhaft sein, einen Teil des Aldehyd-haltigen Stroms 11 (durch eine nicht gezeichnete Leitung) in den ersten Hydroformylierungsreaktor 1 einzubringen.

Die vorliegende Erfindung wird in den nachfolgenden Beispielen beispielhaft beschrieben, ohne dass die Erfmdung, deren Anwendungsbreite sich aus der Beschreibung und den Patentansprüchen ergibt, auf die in den Beispielen angegebenen Ausführungsformen beschränkt sein soll.

### Beispiel 1 Kontinuierlicher 2-Stufen-Prozess, 1. Hydroformylierungsstufe

Eine Hydroformylierung von Olefinen wurde in einer Versuchsanlage kontinuierlich durchgeführt, die im wesentlichen aus einem senkrecht stehenden Hochdruckrohrreaktor (90 mm Durchmesser, 3600 mm Länge) und einem nachgeschalteten mit Raschig-Ringen befüllten Entkobaltungsbehälter (5 l Inhalt) sowie einem Phasentrennbehälter (30 l Inhalt) bestand. Durch mehrere orthogonal zu der Strömungsrichtung eingebaute Lochbleche (5 Lochbleche, das erste auf 1000 mm Reaktorhöhe, die nächsten in 500 mm Abstand) war der Reaktorraum des Hochdruckreaktors kaskadiert. Für die Dosierung der Ausgangsstoffe am unteren Ende des Reaktors wurde eine 3-Stoff-Mischdüse verwendet. Der Reaktorinhalt konnte je nach Bedarf über die installierten Heiz- und Kühlvorrichtungen aufgeheizt oder gekühlt werden.

Vor dem Beginn der Hydroformylierung wurde dem Reaktor 15 l Di-n-buten (aus den Octol-Prozess der OXENO Olefinchemie GmbH) und 3 1 wässriger Kobaltacetat-Lösung (1 Massen-% Kobalt) zugeführt. Nachdem der Reaktor auf die Betriebstemperatur von 175 bis 185 °C gebracht worden war, wurden über die Mischdüse die weiteren Ausgangsstoffe: C₈-Olefin (Din-buten aus dem OXENO Octol-Prozess), wässrige Kobaltacetat-Lösung mit 1 Massen-% Kobalt und das Synthesegas (Volumenverhältnis CO/H₂ = 1 : 1) dem Reaktor kontinuierlich zugeführt.

Folgende Durchsätze wurden eingestellt: 10,0 kg/h Di-n-buten und 0,50kg /h Co-Acetat-Lösung. Der Reaktor wurde mit Synthesegas auf einem konstanten Reaktionsdruck von 27 MPa bei einem Synthesegas-Durchsatz von 5,0 bis 9,5 Nm³/h druckgeregelt. Nach einer Versuchzeit von 5 h wurde ein stationärer Betriebzustand erreicht. Die organische Phase wurde kontinuierlich am Kopf des Reaktors abgezogen, auf 1 bis 1,5 MPa entspannt und anschließend in den Entkobaltungsbehälter geführt. Vor dem Entkobaltungsbehälter wurde der organischen Phase ein Prozesswasser ( 8 kg/h) zugeführt.

In der Entkobaltungsstufe wurde das nun zweiphasige Gemisch bei 140 °C mit 25 Nl/h Luft in Gegenwart von Prozesswasser von Co-Carbonylkomplexen durch Umsetzung zum Kobaltacetat und/oder Kobaltformiat befreit und anschließend in einem nachgeschalteten Trennbehälter getrennt. Die organische weitestgehend kobaltfreie Phase wurde durch Destillation von nicht umgesetzten C₈-Olefinen befreit (siehe Beispiele 2 und 3).

Unter den gewählten Reaktionsbedingungen wurden Di-n-buten-Umsätze von rd. 80 % erzielt. Der Rohproduktaustrag nach der Entkobaltung wies nach einer GC-Analyse folgende Zusammensetzung in Massen-% auf: 19,8 % C₈-Kohlenwasserstoffe davon 4,6 % C₈-Paraffine, 57,6 % Isononanale, 18,3 % Isononanole, 2,7 % Ester (Isononylformiate) und 1,6 % Rückstand. Nach der Entkobaltung wurden in einer nachgeschalteten Destillation wie in Beispiel 2 und 3 beschrieben die C₈-Kohlenwasserstoffe im Rohprodukt von Wertprodukten (Isononanal, Isononanol und Isononylformiate) abgetrennt. Die C₈-Olefin-haltigen Kohlenwasserstoffe wurden als Edukt in der zweiten Oxo-Stufe eingesetzt.

### Beispiele 2 und 3 Destillative Abtrennung von C₈-Kohlenwasserstoffen

Mit dem Ziel C₈-Kohlenwasserstoffe (Olefme und Paraffine) von den Wertprodukten zu trennen, wurden in einer Destillationskolonne in zwei Versuchen batchweise jeweils rd. 250 kg eines Reaktionsproduktes (Produkt der Entkobaltungsstufe) aus dem Beispiel 1 destilliert.

Für die Destillationsversuche wurde eine Batch-Kolonne mit einem Durchmesser von 80 mm, die mit 5 m Sulzer DX Laborpackung gefüllt war, verwendet.

### Bespiel 2 (nicht erfindungsgemäß)

In einem ersten Destillationsversuch bei einer Kopftemperatur von 67 °C, einem Druck von 0,01 MPa und einem Rücklaufverhältnis von 5 wurde über Kopf als Leichtsieder eine praktisch C₉-Aldehyd-freie C₈-Kohlenwasserstoff-Fraktion (46 kg) abgezogen. Nach einer GC-Analyse enthielt diese Fraktion 99,9 Massen-% C₈-Rest-Kohlenwasserstoffe (76,9 Massen-% C₈₋Olefine, 23,0 Massen-% C₈-Paraffine) und 0,1 Massen-% C₉-Aldehyde. Die Sumpffraktion (197 kg) enthielt als Wertprodukte C₉-Aldehyde, C₉-Alkohole und Isononylformiate. Folgende Zusammensetzung der Sumpffraktion wurde durch eine GC-Analyse ermittelt: 71,7 Massen-% Isononanale, 22,7 Massen-% Isononanole, 3,4 Massen-% Ester (Isononylformiate), 2,0 Massen-% Hochsieder und 0,2 Massen-% C₈-Kohlenwasserstoffe. Die C₈-Olefin-haltige Kopffraktion wurde als Edukt für die Hydroformylierung in der zweiten Oxo-Stufe eingesetzt (siehe Beispiel 4).

### Beispiel 3 (erfindungsgemäß)

Bei der Destillation der zweiten 250 kg des Produktes aus der Entkobaltungsstufe wurden durch die Wahl von entsprechenden Destillationsbedingungen (Kopftemperatur 70 °C, Druck 0,01 MPa und Rücklauf von 2) gezielt höhere Gehalte an C₉-Aldehyd (Isononanale) in der C₈₋Kohlenwasserstoff-Fraktion zugelassen. Die über Kopf als Leichtsieder abgezogene Fraktion von rd. 45,5 kg enthielt neben 95,0 Massen-% C₈-Kohlenwasserstoffe rd. 5,0 Massen-% Isononanale. Die C₈-Olefin-haltige Kopffraktion wird als Edukt für die Hydroformylierung in der zweiten Hydroformylierungsstufe eingesetzt (siehe Beispiel 5). Für die Sumpffraktion (196 kg) wurde nach GC-Analyse folgende Zusammensetzung ermittelt: 71,5 Massen-% Isononanale (INA), 23,0 Massen-% Isononanole, 3,3 Massen-% Ester (Isononylformiate), 2,0 Massen-% Hochsieder und 0,2 Massen-% C8-Kohlenwasserstoffe.

### Beispiel 4 (nicht erfindungsgemäß) 2. Hydroformylierungsstufe unter Verwendung von INA-freien C₈-Olefinen

Die Hydroformylierung der durch Destillation gewonnen Olefine aus dem Beispiel 2 wurde wie in Beispiel 1 in einer Versuchsanlage kontinuierlich durchgeführt. Die als Edukt verwendeten C₈-Rest-Kohlenwasserstoffe aus der ersten Oxo-Stufe enthielten neben 77 Massen-% C₈₋Olefine zusätzlich rund 23 Massen-% C₈-Paraffine, die durch unerwünschte Hydrierung der C₈₋Olefine in der ersten Hydroformylierungsstufe gebildet worden waren. Nachdem der Reaktor auf die Betriebstemperatur von 180 bis 195 °C gebracht worden war, wurden über die Mischdüse die Ausgangsstoffe: die C₈-Rest-Kohlenwasserstoffe, die wässrige Kobaltacetat-Lösung mit 1 Massen-% Co und das Synthesegas (Volumenverhältnis CO /H₂ = 1 : 1) dem Reaktor kontinuierlich zugeführt. Vor dem Beginn der Hydroformylierung werden in dem Reaktor die Rest-C₈-Kohlenwasserstoffe und Kobaltacetat-Lösung vorgelegt (Mengen siehe Beispiel 1).

Folgende Durchsätze wurden eingestellt: 5,0 kg/h C₈-Kohlenwasserstoffgemisch und 0,35 kg/h Co-Acetat-Lösung. Der Reaktor wurde mit Synthesegas auf einem konstanten Reaktionsdruck von 27 MPa bei einem Synthesengas-Durchsatz von 2,5 bis 5,5 Nm³/h druckgeregelt. Die organische Phase wird kontinuierlich am Kopf des Reaktors (7,4 kg/h) abgezogen und in den Entkobaltungsbehälter auf 1,0 bis 1,5 MPa entspannt. Vor dem Entkobaltungsbehälter wurde der organischen Phase 4 kg/h Prozesswasser zugeführt.

In der Entkobaltungsstufe wird das im Behälter nun vorliegende zweiphasige Gemisch bei 140 °C in Gegenwart von 15 Nl/h Luft umgesetzt und die organische Phase von Co-Carbonylkomplexen durch Umsetzung zum Kobaltacetat befreit und anschließend in einem nachgeschalteten Trennbehälter von der wässrigen Phase getrennt. Die organische weitestgehend kobaltfreie Phase wurde analysiert. Der Rohproduktaustrag nach der Entkobaltung wies nach einer GC-Analyse folgende Zusammensetzung in Massen-% auf: 29,3 C₈-Kohlenwasserstoffe (6,9 C₈-Olefine, 22,4 C₈-Paraffine), 23,3 Isononanale, 36,9 Isononanole, und 5,3 Ester (Isononylformiate) sowie 5,2 Rückstand. Unter den gewählten Reaktionsbedingungen wurden C₈-Olefin-Umsätze von rd. 88,8 % und Wertproduktausbeuten von 81,9 % erzielt

### Beispiel 5 (erfindungsgemäß) 2. Hydroformylierungsstufe mit INA-haltigen C₈-Olefinen

Die Hydroformylierung der durch Destillation gewonnenen Olefine aus dem Beispiel 3 wird wie in Beispiel 4 beschrieben in einer Versuchsanlage kontinuierlich durchgeführt. Die als Edukt (Kopffraktion aus Beispiel 3) verwendeten C₈-Rest-Kohlenwasserstoffe enthielten neben 73,18 Massen-% C₈-Olefine und 21,86 Massen-% C₈-Paraffine zusätzlich 4,96 Massen-% C₉₋Aldehyde.

Nachdem der Reaktor auf die Betriebstemperatur von 180 bis 195 °C gebracht worden war, wurden über die Mischdüse die Ausgangsstoffe, die C₈-Rest-Kohlenwasserstoffe, die wässrige Kobaltacetat-Lösung mit 1 Gew.-% Co und das Synthesegas (Volumenverhältnis CO/H₂ = 1 : 1) dem Reaktor kontinuierlich zugeführt.

Folgende Durchsätze wurden eingestellt: 5,28 kg/h Destillat aus Beispiel 3 und 0,35 kg/h Co-Acetat-Lösung. Der Reaktor wurde mit Synthesegas auf einem konstanten Reaktionsdruck von 27 MPa bei einem Synthesengas-Durchsatz von 2,5 bis 5,5 Nm³/h druckgeregelt. Die organische Phase wurde kontinuierlich am Kopf des Reaktors(7,6 kg/h) abgezogen und in den Entkobaltungsbehälter auf 1,0 bis 1,5 MPa entspannt. Vor dem Entkobaltungsbehälter wurde der organischen Phase 4 kg/h Prozesswasser zugeführt. In der Entkobaltungsstufe wird die organische Phase bei 140 °C in Gegenwart von 16 Nl/h Luft von Co-Carbonylkomplexen durch Umsetzung zum Kobaltacetat und/oder Kobaltformiat befreit und anschließend in einem nachgeschalteten Trennbehälter von der wässrigen Phase getrennt.

Die organische weitestgehend kobaltfreie Wertprodukt-Phase wurde analysiert. Die mit dem Edukt dem Reaktor zugeführten Isononanal-Mengen wurden bei der Auswertung der Analysenergebnisse (Berechnung von Umsatz und Ausbeute) berücksichtigt. Der Rohproduktaustrag nach der Entkobaltung wies nach einer GC-Analyse folgende Zusammensetzung in Massen-% auf: 26,3 % C₈-Kohlenwasserstoffe (5,2 C₈-Olefine, 21,1 C₈₋Paraffine), 20,8 Isononanale, 41,9 Isononanole, und 5,4 Ester (Isononylformiate) sowie 5,6 % Rückstand. Unter den gewählten Reaktionsbedingungen wurden C₈-Olefine-Umsätze von ca. 91,7 % und Wertproduktausbeuten von 84,3 % erzielt.

Ein Vergleich der C₈-Olefin-Umsätze und Ausbeuten bei der Hydroformylierung von C₈-Olefinen mit (Beispiel 5) und ohne Isononanal (Beispiel 4) im Edukt zeigte, dass durch Einsatz eines C₉-Aldehyd-haltigen Edukts eine nennenswerte Verbesserung der Umsätze und Ausbeuten erreicht wird. Gemäß den vorliegenden Ergebnissen konnte durch Anwendung des erfindungsgemäßen Verfahrens in der zweiten Hydroformylierungsstufe der Umsatz im Vergleich mit Verfahren des Standes der Technik um ca. 2,9 % Punkte und die Ausbeute um ca. 2,4 % Punkte verbessert werden.

## Patentansprüche

1. Verfahren zur katalytischen Hydroformylierung von Olefinen mit 6 bis 24 C-Atomen,
**dadurch gekennzeichnet,**
**dass** die Hydroformylierung in einer oder mehreren Stufe(n) durchgeführt wird, wobei in zumindest einer dieser Stufen, die in Gegenwart eines unmodifizierten Kobaltkomplexkatalysators durchgeführt wird, denen Liganden ausgewählt sind aus Kohlenmonoxid, Wasserstoff, Olefin, Alkylreste, Alkenylreste, Acylreste oder Alkoxyreste, wobei als Liganden keine Verbindungen, die Elemente der fünften Hauptgruppe des Periodensystems der Elemente (N, P, As, Sb; Bi) enthalten, vorhanden sind, als Edukt ein Gemisch eingesetzt wird, welches zumindest ein Olefin und ein Aldehyd mit 7 bis 25 C-Atomen aufweist, wobei das molare Verhältnis von Aldehyd zu Olefin von 0,005 zu 1 bis 0,2 zu 1 beträgt.

2. Verfahren nach Anspruch 1,
**dadurch gekennzeichnet,**
**dass** das molare Verhältnis von Aldehyd zu Olefin von 0,01 zu 1 bis 0,07 zu 1 beträgt.

3. Verfahren nach Anspruch 1 oder 2,
**dadurch gekennzeichnet,**
**dass** die Hydroformylierung in einer Stufe durchgeführt wird.

4. Verfahren nach Anspruch 1 oder 2,
**dadurch gekennzeichnet,**
**dass** das Verfahren in mindestens zwei Hydroformylierungsstufen durchgeführt wird.

5. Verfahren nach Anspruch 4,
**dadurch gekennzeichnet,**
**dass** in mindestens einer Hydroformylierungsstufe ein modifizierter oder unmodifizierter Rhodiumkomplex als Katalysator eingesetzt wird.

6. Verfahren nach Anspruch 4,
**dadurch gekennzeichnet,**
**dass** in allen Hydroformylierungsstufen Kobaltkomplexe als Katalysatoren eingesetzt werden.

7. Verfahren nach Anspruch 6,
**dadurch gekennzeichnet,**
**dass** in allen Hydroformylierungsstufen unmodifizierte Kobaltkomplexe als Katalysatoren eingesetzt werden.

8. Verfahren nach Anspruch 6 oder 7,
**dadurch gekennzeichnet,**
**dass** in allen Hydroformylierungsstufen in denen Kobaltkomplexe als Katalysatoren eingesetzt werden, als Edukt ein Gemisch eingesetzt wird, welches zumindest ein Olefin und ein Aldehyd aufweist.

9. Verfahren nach einem der Ansprüche 1 bis 8,
**dadurch gekennzeichnet,**
**dass** in zumindest einer Hydroformylierungsstufe unmodifizierte Kobaltkomplexe eingesetzt werden, die gleichzeitig mit der Hydroformylierung durch Umsetzung einer wässrigen Kobaltsalzlösung mit Synthesegas in der Hydroformylierungsstufe erzeugt werden.

10. Verfahren nach einem der Ansprüche 1 bis 9,
**dadurch gekennzeichnet,**
**dass** ein Destillat, das Aldehyde und Olefine enthält und das aus einem Hydroformylierungsgemisch abgetrennt wurde, zur Herstellung des Eduktgemisches aus Aldehyd und Olefin oder direkt als Eduktgemisch eingesetzt wird.

11. Verfahren nach einem der Ansprüche 1 bis 10,
**dadurch gekennzeichnet,**
**dass** ein Hydroformylierungsgemisch zur Herstellung des Eduktgemisches aus Aldehyd und Olefin eingesetzt wird.

12. Verfahren nach einem der Ansprüche 1 bis 11,
**dadurch gekennzeichnet,**
**dass** ein Destillat, das Aldehyde und Olefine enthält und das aus einem Hydroformylierungsgemisch abgetrennt wurde, und ein Hydroformylierungsgemisch zur Herstellung des Eduktgemisches aus Aldehyd und Olefin eingesetzt werden.

13. Verfahren nach Anspruch 11 oder 12,
**dadurch gekennzeichnet,**
**dass** ein entkatalysiertes Hydroformylierungsgemisch eingesetzt wird.

14. Verfahren nach einem der Ansprüche 1 bis 13,
**dadurch gekennzeichnet,**
**dass** zumindest ein Teil des Hydroformylierungsgemisches der letzten Hydroformylierungsstufe hydriert wird.

15. Verfahren nach einem der Ansprüche 1 bis 14,
**dadurch gekennzeichnet,**
**dass** aus dem flüssigen Hydroformylierungsgemisch einer oder mehrere Hydroformylierungsstufen nach Entfernen des Katalysators die nicht umgesetzten Olefine abgetrennt werden.

16. Verfahren nach Anspruch 15,
**dadurch gekennzeichnet,**
**dass** ein Teil der abgetrennten Olefine in eine oder mehrere Hydroformylierungsstufen zurückgeführt wird.

## Claims

1. Process for the catalytic hydroformylation of olefins having from 6 to 24 carbon atoms, **characterized in that** the hydroformylation is carried out in one or more stage (s) and a mixture comprising at least one olefin and an aldehyde having from 7 to 25 carbon atoms in a molar ratio of aldehyde to olefin of from 0.005:1 to 0.2:1 is used as feed in at least one of these stages which is carried out in the presence of an unmodified cobalt complex as catalyst, whose ligands are selected from among carbon monoxide, hydrogen, olefin, alkyl radicals, alkenyl radicals, acyl radicals and alkoxy radicals, and no compounds containing elements of the fifth main group of the Periodic Table of Elements (N, P, As, Sb, Bi) are present as ligands.

2. Process according to Claim 1, **characterized in that** the molar ratio of aldehyde to olefin is from 0.01:1 to 0.07:1.

3. Process according to claim 1 or 2, **characterized in that** the hydroformylation is carried out in one stage.

4. Process according to Claim 1 or 2 **characterized in that** the process is carried out in at least two hydroformylation stages.

5. Process according to Claim 4, **characterized in that** a modified or unmodified rhodium complex is used as catalyst in at least one hydroformylation stage.

6. Process according to Claim 4, **characterized in that** cobalt complexes are used as catalysts in all hydroformylation stages.

7. Process according to Claim 6, **characterized in that** unmodified cobalt complexes are used as catalysts in all hydroformylation stages.

8. Process according to Claim 6 or 7, **characterized in that** a mixture comprising at least one olefin and an aldehyde is used as feed in all hydroformylation stages in which cobalt complexes are used as catalysts.

9. Process according to any of Claims 1 to 8, **characterized in that** unmodified cobalt complexes which are generated simultaneously with the hydroformylation by reaction of an aqueous cobalt salt solution with synthesis gas in the hydroformylation stage are used in at least one hydroformylation stage.

10. Process according to any of Claims 1 to 9, **characterized in that** a distillate which comprises aldehydes and olefins and has been separated off from a hydroformylation mixture is used for producing the feed mixture of aldehyde and olefin or directly as feed mixture.

11. Process according to any of Claims 1 to 10, **characterized in that** a hydroformylation mixture is used for producing the feed mixture of aldehyde and olefin.

12. Process according to any of Claims 1 to 11, **characterized in that** a distillate which comprises aldehydes and olefins and has been separated off from a hydroformylation mixture and a hydroformylation mixture are used for producing the feed mixture of aldehyde and olefin.

13. Process according to Claim 11 or 12, **characterized in that** a decatalyzed hydroformylation mixture is used.

14. Process according to any of Claims 1 to 13, **characterized in that** at least part of the hydroformylation mixture from the last hydroformylation stage is hydrogenated.

15. Process according to any of Claims 1 to 14, **characterized in that** the unreacted olefins are separated off from the liquid hydroformylation mixture from one or more hydroformylation stages after removal of the catalyst.

16. Process according to Claim 15, **characterized in that** part of the olefins which have been separated off is recirculated to one or more hydroformylation stages.

## Revendications

1. Procédé pour l'hydroformylation catalytique d'oléfines comprenant 6 à 24 atomes de carbone, **caractérisé en ce que** l'hydroformylation est réalisée en une ou plusieurs étapes, où dans au moins une de ces étapes, qui est réalisée en présence d'un catalyseur complexe du cobalt non modifié, dont les ligands sont choisis parmi le monoxyde de carbone, l'hydrogène, une oléfine, des radicaux alkyle, des radicaux alcényle, des radicaux acyle ou des radicaux alcoxy, où il n'existe pas, comme ligands, de composés qui contiennent des éléments du cinquième groupe principal du système périodique des éléments (N, P, As, Sb, Bi), on utilise comme produit de départ un mélange, qui présente au moins une oléfine et un aldéhyde comprenant 7 à 25 atomes de carbone, le rapport molaire d'aldéhyde à oléfine valant de 0,005:1 à 0,2:1.

2. Procédé selon la revendication 1, **caractérisé en ce que** le rapport molaire d'aldéhyde à oléfine est de 0,01:1 à 0,07:1.

3. Procédé selon la revendication 1 ou 2, **caractérisé en ce que** l'hydroformylation est réalisée en une étape.

4. Procédé selon la revendication 1 ou 2, **caractérisé en ce que** le procédé est réalisé en au moins deux étapes d'hydroformylation.

5. Procédé selon la revendication 4, **caractérisé en ce qu'**on utilise, dans au moins une étape d'hydroformylation, un complexe du rhodium modifié ou non modifié comme catalyseur.

6. Procédé selon la revendication 4, **caractérisé en ce qu'**on utilise, dans toutes les étapes d'hydroformylation, des complexes du cobalt comme catalyseurs.

7. Procédé selon la revendication 6, **caractérisé en ce qu'**on utilise, dans toutes les étapes d'hydroformylation, des complexes du cobalt non modifiés comme catalyseurs.

8. Procédé selon la revendication 6 ou 7, **caractérisé en ce qu'**on utilise, dans toutes les étapes d'hydroformylation dans lesquelles on utilise des complexes de cobalt comme catalyseurs, comme produit de départ, un mélange qui présente au moins une oléfine et un aldéhyde.

9. Procédé selon l'une quelconque des revendications 1 à 8, **caractérisé en ce qu'**on utilise, dans au moins une étape d'hydroformylation, des complexes du cobalt non modifiés, qui sont générés en même temps que l'hydroformylation, par transformation d'une solution aqueuse de sel de cobalt avec du gaz de synthèse lors de l'étape d'hydroformylation.

10. Procédé selon l'une quelconque des revendications 1 à 9, **caractérisé en ce qu'**on utilise un distillat, contenant des aldéhydes et des oléfines et qui a été séparé d'un mélange d'hydroformylation, pour la préparation du mélange de produits de départ constitué par l'aldéhyde et l'oléfine ou directement en tant que mélange de produits de départ.

11. Procédé selon l'une quelconque des revendications 1 à 10, **caractérisé en ce qu'**on utilise un mélange d'hydroformylation pour la préparation du mélange de produits de départ constitué par l'aldéhyde et l'oléfine.

12. Procédé selon l'une quelconque des revendications 1 à 11, **caractérisé en ce qu'**on utilise un distillat, contenant des aldéhydes et des oléfines et qui a été séparé d'un mélange d'hydroformylation, et un mélange d'hydroformylation pour la préparation du mélange de produits de départ constitué par l'aldéhyde et l'oléfine.

13. Procédé selon la revendication 11 ou 12, **caractérisé en ce qu'**on utilise un mélange d'hydroformylation décatalysé.

14. Procédé selon l'une quelconque des revendications 1 à 13, **caractérisé en ce qu'**au moins une partie du mélange d'hydroformylation de la dernière étape d'hydroformylation est hydrogénée.

15. Procédé selon l'une quelconque des revendications 1 à 14, **caractérisé en ce qu'**on sépare du mélange d'hydroformylation liquide provenant d'une ou de plusieurs étapes d'hydroformylation, après l'élimination du catalyseur, les oléfines non transformées.

16. Procédé selon la revendication 15, **caractérisé en ce qu'**une partie des oléfines séparées est recyclée dans une ou plusieurs étapes d'hydroformylation.
